(19) Europäisches Patentamt
European Patent Office
Office européen des brevets

(11) EP 3 730 306 B1

(12) # EUROPÄISCHE PATENTSCHRIFT

(45) Veröffentlichungstag und Bekanntmachung des Hinweises auf die Patenterteilung:
**16.03.2022 Patentblatt 2022/11**

(51) Internationale Patentklassifikation (IPC):
***B41M 5/333*** *(2006.01)* ***C07C 311/57*** *(2006.01)*
***C07C 311/60*** *(2006.01)* *B41M 5/327* *(2006.01)*

(21) Anmeldenummer: **20179846.9**

(52) Gemeinsame Patentklassifikation (CPC):
**B41M 5/3333; C07C 311/60;** B41M 5/3275

(22) Anmeldetag: **17.05.2018**

(54) **NICHT-PHENOLISCHER FARBENTWICKLER UND WÄRMEEMPFINDLICHES AUFZEICHNUNGSMATERIAL**

NON-PHENOLIC COLOUR DEVELOPER AND HEAT-SENSITIVE RECORDING MATERIAL

DÉVELOPPEUR DE COULEUR NON PHÉNOLIQUE ET MATÉRIEL D'ENREGISTREMENT THERMOSENSIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2017 DE 102017111439**

(43) Veröffentlichungstag der Anmeldung:
**28.10.2020 Patentblatt 2020/44**

(62) Dokumentnummer(n) der früheren Anmeldung(en) nach Art. 76 EPÜ:
**18726113.6 / 3 630 496**

(73) Patentinhaber: **Papierfabrik August Koehler SE**
**77704 Oberkirch (DE)**

(72) Erfinder:
- **HORN, Michael**
  **77654 Offenburg (DE)**
- **STALLING, Timo**
  **77767 Appenweier (DE)**
- **STEPPAT, Maren**
  **77704 Oberkirch (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(56) Entgegenhaltungen:
**EP-A1- 0 610 653** **EP-A1- 0 620 122**
**WO-A1-01/72527** **US-A1- 2006 004 197**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 730 306 B1

## Beschreibung

Die Erfindung betrifft einen Farbentwickler, ein wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, sowie ein Verfahren zu dessen Herstellung,

Wärmeempfindliche Aufzeichnungsmaterialien für die Thermodirekt-Druckanwendung, die eine auf einem Trägersubstrat aufgebrachte wärmeempfindliche farbbildende Schicht (Thermoreaktionsschicht) aufweisen, sind seit langem bekannt. In der wärmeempfindlichen farbbildenden Schicht liegen üblicherweise ein Farbbildner und ein Farbentwickler vor, die unter Wärmeeinwirkung miteinander reagieren und so zu einer Farbentwicklung führen. Häufig kommen dabei (Bis)phenolische Farbentwickler zum Einsatz. Ebenfalls bekannt sind wärmeempfindliche Aufzeichnungsmaterialien, die in der wärmeempfindlichen farbbildenden Schicht einen nicht-phenolischen Farbentwickler enthalten. Diese wurden entwickelt, um die Beständigkeit des Schriftbildes zu verbessern, insbesondere auch dann, wenn das bedruckte wärmeempfindliche Aufzeichnungsmaterial über längere Zeit gelagert wird oder mit hydrophoben Stoffen, wie weichmacherhaltigen Materialien oder Ölen, in Kontakt kommt. Insbesondere vor dem Hintergrund der öffentlichen Diskussionen über das toxische Potenzial (bis)phenolischer Chemikalien ist das Interesse an nicht-phenolischen Farbentwicklern stark angestiegen. Hierbei war es Ziel, die toxikologischen Nachteile der phenolischen Farbentwickler zu vermeiden, allerdings sollten die technischen Leistungseigenschaften, die mit phenolischen Farbentwicklern erzielt werden können, zumindest beibehalten, vorzugsweise aber verbessert werden.

Die US 2006/004197 A1 offenbart Verbindungen der folgenden Formel

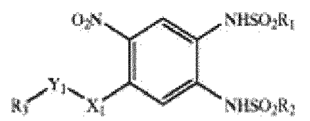

,

wobei R1 p-$(C_6H_4)CH_3$ oder $CH_3$ ist;
wobei $R_2$ p-$(C_6H_4)CH_3$ oder $CH_3$ ist;
wobei $R_3$ F, Cl, p-$(C_6H_4)OCH_3$, o-$(C_6H_4)OCH_3$, m-$(C_6H_4)OCH_3$, p-$(C_6H_4)OH$, p-$(C_6H_4)Cl$, o-$(C_6H_4)Cl$, m-$(C_6H_4)Cl$, p-$(C_6H_4)F$, p-$(C_6H_4)CH_3$, o-$(C_6H_4)CH_3$, m-$(C_6H_4)CH_3$, 3,5-$(C_6H_3)(CH_3)_2$, 2,6-$(C_6H_3)(CH_3)_2$, o-$(C_6H_4)[CH(CH_3)_2]$, $C_6H_5$, 2-Furyl, Morpholin-4-yl, n-Me-piperazin-1-yl, t
Thiomorpholin-4-yl, 3-Pyridyl, 2-Pyridyl, Cyclohexyl, Cyclohexyl-1-ol, or 5-Me-pyrazol-4-yl ist;
wobei $X_1$ S, NH oder O ist; und
wobei $Y_1$ $(CH_2)_n$ ist, wobei n im Bereich von 1 bis 3 liegt.

Die WO 01/72527 A1 offenbart ein wärmeempfindliches Aufzeichnungsmaterial, das einen Farbentwickler der folgenden Formel umfasst

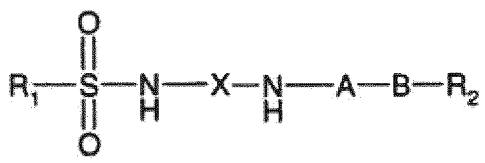

,

wobei $R_1$ ein unsubstituierter oder substituierter Phenyl-, Naphtyl- oder $C_{1-20}$alkylRest ist,
X ausgewählt ist aus den folgenden Formeln

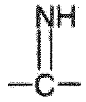
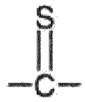
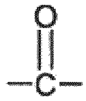

A ein unsubstituierter oder substituierter Phenyl-, Naphtyl- oder $C_{1-20}$Alkyl-Rest, oder eine unsubstituierte oder substituierte heterocyclische Gruppe ist,
B eine Linkergruppe der Formel -O-$SO_2$-, -$SO_2$-O-, -NH-$SO_2$-, -$SO_2$-NH-. -S-$SO_2$-,-O-CO-, -O-CO-NH-, -NH-CO, -NH-CO-O-, -S-CO-NH-, -S-CS-NH-, -CO-NH-$SO_2$, -O-CO-NH-$SO_2$-, -NH=CH, -CO-NH-CO-, -S-, -CO-, -O-, -$SO_2$-NH-CO-,

-O-CO-O und-O-PO-$(OR_2)_2$ ist, wobei $R_2$ ein unsubstituierter oder substituierter Aryl-, Benzyl- oder $C_1$-$C_{20}$alky-Rest ist, mit der Ausnahme, dass, wenn B keine Linkergruppe der Formel -O-$SO_2$- ist, $R_2$ ein unsubstituierter oder substituierter Phenyl-, Naphtyl- oder $C_{1-8}$Alkyl-Rest ist.

Der Stand der Technik zu nicht-phenolischen Farbentwicklern lässt trotz der großen chemischen Diversität dieser Stoffe gemeinsame strukturelle Merkmale erkennen.

So ist eine 1,3-disubstituierte Ureido-Substruktur (Y-NH-CO-NH-Z) ein gemeinsames Merkmal zahlreicher nicht-phenolischer Farbentwickler. Durch passende Wahl der Gruppen Y und Z können die für die Eignung als Farbentwickler relevanten funktionellen Eigenschaften moduliert werden.

Weit verbreitet sind Farbentwickler mit Sulfonyl-Harnstoff-Strukturen (-$SO_2$-NH-CO-NH-), da diese relativ leicht herstellbar sind und die mit ihnen hergestellten wärmeempfindlichen Aufzeichnungsmaterialien gute anwendungstechnische Eigenschaften aufweisen.

Die EP 0 526 072 A1 offenbart Farbentwickler aus der Klasse der aromatischen Sulfonyl-(Thio)Harnstoffverbindungen der Formel

$$Ar'-SO_2-NH-C(X)-NH-Ar,$$

wobei X = O oder S ist und Ar und Ar' aromatische Reste sind.

Mit diesen Farbentwicklern können wärmeempfindliche Aufzeichnungsmaterialien erhalten werden, die sich durch eine verbesserte Bildbeständigkeit auszeichnen. Ferner weisen die auf diesen Farbentwicklern basierenden wärmeempfindlichen Aufzeichnungsmaterialien eine brauchbare thermische Druckempfindlichkeit bei guter Oberflächenweiße auf, so dass es bei entsprechender Gestaltung der Rezeptur der wärmeempfindlichen farbbildenden Schicht vergleichsweise leicht möglich ist, hohe Druckdichten unter Verwendung handelsüblicher Thermodrucker zu erzeugen.

Die WO 0 035 679 A1 offenbart aromatische und heteroaromatische Sulfonyl-(Thio)Harnstoffverbindungen (X = S oder O) und/oder Sulfonyl-Guanidine (X = NH) der obigen Formel, wobei Ar durch eine zweiwertige Linkergruppe an weitere aromatische Gruppen geknüpft ist. Ein in der Praxis verbreiteter nicht-phenolischer Farbentwickler aus dieser Klasse, 4-Methyl-*N*-(((3-(((4-methylphenyl)sulfonyl)oxy)phenyl)amino)carbonyl)benzolsulfonamid (Handelsname Pergafast 201®, BASF), zeichnet sich durch Ausgewogenheit der anwendungstechnischen Eigenschaften der mit diesem Farbentwickler hergestellten wärmeempfindlichen Aufzeichnungsmaterialien aus. Insbesondere besitzen diese eine gute dynamische Ansprechempfindlichkeit und eine akzeptable Beständigkeit des Ausdrucks gegenüber hydrophoben Stoffen.

Auch über eine zwei- oder mehrwertige Linkergruppe A verbundene Sulfonylharnstoffeinheiten, z. B. Bis-Sulfonylharnstoffverbindungen, der Formel

$$(Ar-SO_2-NH-C(O)-NH-)_2A,$$

wobei Ar ein aromatischer Rest ist, sind vielfach als Farbentwickler beschrieben worden (siehe EP 0 535 887 A1, EP 0 542 556 A1, EP 0 604 832 B1, EP 0 620 122 A1 und EP 1 044 824 A2).

In der Praxis hat sich vor allem *N,N'*-(Methylenbis(4,1-phenylenimino-carbony))bis(4-methyl-benzolsulfonamid) (B-TUM) durchgesetzt (A = $CH_2$, Ar = 4-Methyl-phenyl).

Die Kombination einer Sulfonyl-Harnstoff-Struktur mit einer *N*-Sulfonyl-(Thio)Ur-ethan-Gruppe der Formel

$$(Ar-SO_2-NH-C(O)-NH-)_n(Ar-SO_2-NH-C(O)-X-)_mA,$$

wobei Ar ein aromatischer Rest, A eine (m+n)-wertige organische LinkerGruppe und X = O oder S ist, ist Gegenstand der JP H 0 664 335.

Für die mit diesen Farbentwicklern hergestellten wärmeempfindlichen Aufzeichnungsmaterialien wird eine gesteigerte Beständigkeit des Schriftbildes gegenüber hydrophoben Agenzien beschrieben. Allerdings ist der synthetische Zugang zu diesen Farbentwicklern insbesondere dann problematisch, wenn chemisch einheitliche Stoffe erwünscht sind.

Die JP H 0 958 242 kombiniert Sulfonylharnstoff-Substrukturen mit primären Sulfonamid-Gruppen, um Farbentwickler der Formel

$$R-SO_2-NH-C(X)-NH-C_6H_4-SO_2-NH_2$$

zu erhalten.

Die JP H 11-263067 offenbart Farbentwickler aus über eine aromatische Linkereinheit verbundene (Thio)Harnstoff- und Sulfonyl-(Thio)Harnstoff-Substrukturen der Formel

$$Ar^1-NH-C(X)-NH-A-SO_2-NH-C(X)-NH-Ar^2,$$

wobei X = O oder S ist und $Ar^1$ und $Ar^2$ aromatische Reste sind.

Den auf der Sulfonylharnstoff-Chemie basierenden mit nicht-phenolischen Farbentwicklern erhaltenen wärmeempfindlichen Aufzeichnungsmaterialien ist gemein, dass sie in manchen anwendungsrelevanten Eigenschaften gute Leistungen zeigen, in anderen jedoch Schwächen offenbaren.

So geht häufig beispielsweise eine hohe Beständigkeit der Schrift gegenüber hydrophoben Stoffen mit einer bescheidenen Ansprechempfindlichkeit (dynamische Sensitivität) im Thermodrucker einher, welche effektiv nur durch Einsatz großer Mengen zum Teil ganz spezieller Schmelzhilfsmittel (spezielle thermische Lösungsmittel oder spezielle Sensibilisierungsmittel) verbessert werden kann.

Andererseits erreicht man mit bestimmten nicht-phenolischen Farbentwicklern relativ leicht hohe dynamische Sensitivitätswerte, wobei aber die Beständigkeit des Schriftbildes bescheiden ist. Durch Zuhilfenahme von Alterungsschutzmitteln (Stabilisatoren) kann dieser Mangel behoben werden, allerdings auf Kosten einer komplexeren und teureren Rezeptur der Aufzeichnungsschicht.

Aufgabe der vorliegenden Erfindung ist es daher, vorstehend geschilderte Nachteile des Standes der Technik zu beheben. Insbesondere besteht die Aufgabe der vorliegenden Erfindung darin, ein wärmeempfindliches Aufzeichnungsmaterial bereitzustellen, welches eine hohe dynamische Sensitivität (hohe Ansprechempfindlichkeit im Thermodrucker) besitzt und hinsichtlich anderer anwendungstechnisch relevanter Leistungsmerkmale mindestens das Niveau der auf nicht-phenolischen Farbentwicklern des Standes der Technik basierenden Aufzeichnungsmaterialien erreicht, ohne auf spezielle Rezepturbestandteile in der wärmeempfindlichen Funktionsschicht wie Alterungsschutzmittel oder spezielle Schmelzhilfsmittel mit eingeschränkter Verfügbarkeit und hohem Preis angewiesen zu sein. Eine vornehmliche Aufgabe der Erfindung war es, Farbentwickler bereitzustellen, die die Ausbildung einer hohen Druckdichte des Bildes ermöglichen, ohne unerwünschte Auswirkungen auf die Starttemperatur (statische Sensitivität) des Aufzeichnungsmaterials mit sich zu bringen. Gleichzeitig sollte eine gute Beständigkeit des Schriftbildes des wärmeempfindlichen Aufzeichnungsmaterials insbesondere gegenüber hydrophoben Agenzien im Vergleich zum Stand der Technik erreicht werden.

Erfindungsgemäß wird diese Aufgabe durch den Einsatz einer Verbindung nach Anspruch 1 in einem wärmeempfindlichen Aufzeichnungsmaterial nach Anspruch 9 gelöst.

Überraschenderweise hat es sich gezeigt, dass es möglich ist, mit Farbentwicklern des spezifischen Substitutionsmusters der Formel (I) wärmeempfindliche Aufzeichnungsmaterialien zu erhalten, welche sich durch eine hohe dynamische Sensitivität auszeichnen, die mit vergleichbaren Rezepturen für ein wärmeempfindliches Aufzeichnungsmaterial mit anderen Substitutionsmustern des Farbentwicklers derselben chemischen Verbindungsklasse nicht darstellbar sind. Die mit den erfindungsgemäßen Farbentwicklern hergestellten Aufzeichnungsmaterialien weisen auch eine gute Beständigkeit des Schriftbildes gegenüber hydrophoben Agenzien auf, welche zumindest derjenigen des Standes der Technik entspricht.

Die Verbindung nach Anspruch 1 besitzt die Formel (I),

$$(Ar^1\text{-}SO_2\text{-}NH\text{-})_m\text{-}Y\text{-}(\text{-}NH\text{-}C(O)\text{-}NH\text{-}SO_2\text{-}Ar^2)_n, \qquad (I),$$

wobei $Ar^1$ ein unsubstituierter oder substituierter aromatischer Rest, $Ar^2$ ein unsubstituierter oder substituierter Phenyl-Rest, Y eine (m+n)-fach substituierte Benzol- oder Naphthalin-Gruppe und Y derart substituiert ist, dass mindestens eine $Ar^2$-$SO_2$-NH-C(O)-NH-Gruppe in ortho-Position, d.h. in 1,2-Stellung, zu mindestens einer $Ar^1$-$SO_2$-NH-Gruppe steht.

Vorzugsweise ist m gleich 1 und n größer oder gleich 1.

Vorzugsweise ist n gleich 1 und m 1 oder 2.

Vorzugsweise ist $Ar^1$ ein unsubstituierter oder substituierter Phenyl- oder ein unsubstituierter oder substituierter 2-Naphthyl-Rest.

Besonders bevorzugt ist $Ar^1$ ein unsubstituierter Phenyl- oder ein einfach substituierter Phenyl-Rest.

Vorzugsweise ist der einfach substituierte Phenyl-Rest mit einem $C_1$-$C_5$-Alkyl-, einem Alkenyl-, einem Alkinyl-, einem Benzyl-, einem RO-, einem Halogen-, einem Formyl-, einem ROC-, einem $RO_2C$-, einem CN-, einem $NO_2$-, einem R-$SO_2$O-, einem RO-$SO_2$-, einem R-NH-$SO_2$-, einem R-$SO_2$NH-, einem R-NH-CO-NH-, einem R-$SO_2$-NH-CO-NH-, einem R-NH-CO-NH-$SO_2$- oder einem R-CO-NH-Rest substituiert, wobei R ein $C_1$-$C_5$-Alkyl-, ein Alkenyl-, ein Alkinyl-, ein Phenyl-, ein Tolyl- oder ein BenzylRest, bevorzugt ein Phenyl- oder p-Tolyl-Rest ist.

Bevorzugte Substituenten sind $C_1$-$C_5$-Alkyl-, RO-, Halogen-, $RO_2C$-, R-$SO_2$O-, R-NH-CO-NH- und R-$SO_2$-NH-CO-NH-Reste.

Vorzugsweise ist $Ar^2$ ein unsubstituierter Phenyl- oder ein einfach substituierter Phenyl-Rest, insbesondere ein mit einem $C_1$-$C_4$-Alkyl-Rest, besonders bevorzugt mit einem Methyl-Rest substituierter Phenyl-Rest.

In einer besonders bevorzugten Ausführungsform ist $Ar^1$ ein unsubstituierter Phenyl-Rest oder ein einfach substituierter Phenyl-Rest, $Ar^2$ ein unsubstituierter Phenyl-Rest oder ein einfach substituierter Phenyl-Rest und Y eine (m+n)-fach substituierte Benzol-Gruppe.

Besonders bevorzugte Verbindungen der Formel (I) sind in folgender Tabelle 1 dargestellt.

Tabelle 1: Bevorzugte Verbindungen der Formel (I) mit den angegebenen Bedeutungen für die Y-Gruppe, den $Ar^1$-Rest, den $Ar^2$-Rest, m und n (R= wie vorstehend erwähnt)

| | Y | $Ar^1$ | $Ar^2$ | m | n |
|---|---|---|---|---|---|
| **I** | Phenylen- | Phenyl- | Phenyl- | 1 | 1 |
| **II, XIV, XVII** | Phenylen- | Phenyl- | $C_1$-$C_4$-Alkyl substituierter Phenyl- | 1 | 1 |
| **III, XV, XVIII** | Phenylen- | $C_1$-$C_5$-Alkyl substituierter Phenyl- | Phenyl- | 1 | 1 |
| **IV, V, VI, XVI, XIX** | Phenylen- | $C_1$-$C_5$-Alkyl substituierter Phenyl- | $C_1$-$C_4$-Alkyl substituierter Phenyl- | 1 | 1 |
| **VII** | Phenylen- | RO-substituierter Phenyl- | $C_1$-$C_4$-Alkyl substituierter Phenyl- | 1 | 1 |
| **VIII** | Phenylen- | Halogen-substituierter Phenyl- | $C_1$-$C_4$-Alkyl substituierter Phenyl- | 1 | 1 |
| **IX** | Phenylen- | R-CO-NH-substituierter Phenyl- | $C_1$-$C_4$-Alkyl substituierter Phenyl- | 1 | 1 |
| **X** | Phenylen- | Nitro-substituierter Phenyl- | $C_1$-$C_4$-Alkyl substituierter Phenyl- | 1 | 1 |
| **XI** | Phenylen- | $RO_2C$-substituierter Phenyl- | $C_1$-$C_4$-Alkyl substituierter Phenyl- | 1 | 1 |
| **XII** | Phenylen- | Naphthyl- | $C_1$-$C_4$-Alkyl substituierter Phenyl- | 1 | 1 |
| **XIII** | Phenylen- | Benzyl- | $C_1$-$C_4$-Alkyl substituierter Phenyl- | 1 | 1 |
| **XXI** | Dreifachsubstituierte Benzol- | $C_1$-$C_5$-Alkyl substituierter Phenyl- | Phenyl- | 1 | 2 |
| **XX, XXII, XXIII, XXIV** | Dreifachsubstituierte Benzol- | $C_1$-$C_5$-Alkylsubstituierter Phenyl- | $C_1$-$C_4$Alkyl substituierter Phenyi- | 1 | 2 |

Die Herstellung der erfindungsgemäßen Verbindung der Formel (I) kann nach an sich bekannten Methoden erfolgen.

Reaktionsschema 1 veranschaulicht einen möglichen Syntheseweg für die erfindungsgemäße Verbindung der Formel (I) am Beispiel der Verbindungen **I** bis **XIX** (siehe Tabelle 2).

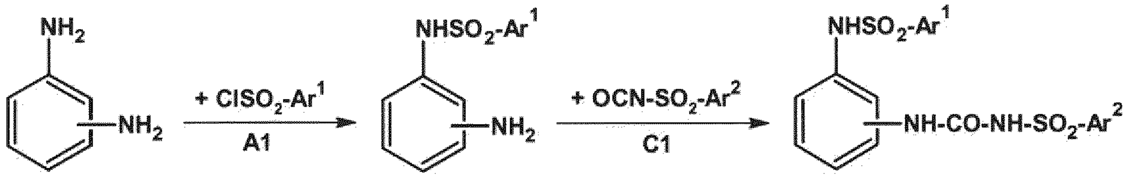


I-XIX

Reaktionsschema 1 ($Ar^1$, $Ar^2$: siehe Tabelle 2)

Reaktionsschema 2 veranschaulicht einen möglichen Syntheseweg für die erfindungsgemäße Verbindung der Formel (I) am Beispiel der Verbindungen **XX** bis **XXIV** (siehe Tabelle 2).

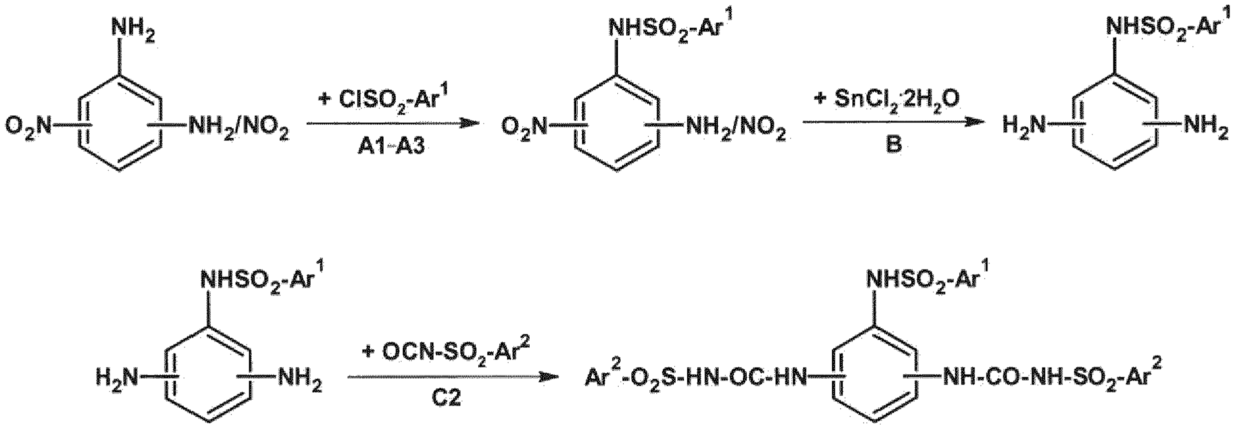


XX-XXIV

Reaktionsschema 2 ($Ar^1$, $Ar^2$: siehe Tabelle 2)

Die unter die erfindungsgemäße Verbindung der Formel (I) fallende Verbindung **XX** (siehe Tabelle 2) kann ausgehend von 2,6-Dinitroanilin, das zunächst gemäß nachfolgendem Reaktionsschema 3 in 1,2-Diamino-3-nitrobenzol (V. Milata, J. Salon, Org. Prep. Proceed. Int., 31 (3), 347 (1999)) und dann nach den beschriebenen Methoden in das Endprodukt überführt wird, hergestellt werden.

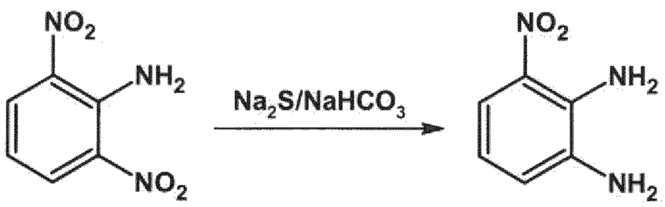


Reaktionsschema 3

Die im Zusammenhang mit der Verbindung der Formel (I) aufgeführten bevorzugten Ausführungsformen gelten ebenfalls für das Verfahren zu dessen Herstellung.

Wie vorstehend bereits erwähnt, betrifft die vorliegende Erfindung auch ein wärmeempfindliches Aufzeichnungsmaterial, umfassend ein Trägersubstrat sowie eine mindestens einen Farbbildner und einen phenolfreien Farbentwickler enthaltende wärmeempfindliche farbbildende Schicht, wobei der mindestens eine phenolfreie Farbentwickler

die Verbindung der vorstehenden beschriebenen Formel (I) ist.

Die Verbindung der Formel (I) liegt vorzugsweise in einer Menge von etwa 3 bis etwa 35 Gew.-%, besonders bevorzugt in einer Menge von etwa 10 bis etwa 25 Gew.-%, bezogen auf den gesamten Feststoffgehalt der wärmeempfindlichen Schicht, vor.

Die Auswahl des Trägersubstrates ist nicht kritisch. Allerdings ist es bevorzugt, als Trägersubstrat Papier, synthetisches Papier und/oder eine Kunststoff-Folie einzusetzen.

Gegebenenfalls liegt zwischen dem Trägersubstrat und der wärmeempfindlichen Schicht mindestens eine weitere Zwischenschicht vor, wobei dieser die Aufgabe zukommt, die Oberflächenglätte des Trägersubstrats für die wärmeempfindliche Schicht zu verbessern und eine Wärmebarriere zwischen Trägersubstrat und der wärmeempfindlichen Schicht zu gewährleisten. Vorzugsweise kommen in dieser Zwischenschicht organische Hohlkugelpigmente und/oder kalzinierte Kaoline zum Einsatz. Auch kann mindestens eine Schutzschicht und/oder mindestens eine die Bedruckbarkeit begünstigende Schicht im erfindungsgemäßen wärmeempfindlichen Aufzeichnungsmaterial vorliegen, wobei diese Schichten auf der Vorder- oder Rückseite des Substrats aufgebracht werden können.

Hinsichtlich der Wahl des Farbbildners unterliegt die vorliegende Erfindung ebenfalls keinen wesentlichen Einschränkungen. Bevorzugt ist der Farbbildner jedoch ein Farbstoff vom Triphenylmethan-, Fluoran-, Azaphthalid- und/oder Fluoren-Typ. Ein ganz besonders bevorzugter Farbbildner ist ein Farbstoff vom Fluoran-Typ, da dieser dank der Verfügbarkeit und der ausgewogenen anwendungsbezogenen Eigenschaften die Bereitstellung eines Aufzeichnungsmaterials mit einem attraktiven Preis-Leistungsverhältnis ermöglicht.

Besonders bevorzugte Farbstoffe vom Fluoran-Typ sind:

3-Diethylami no-6-methyl-7-anilinofluoran,
3-(*N*-ethyt-*N*-p-toiudinamino)-6-methyl-7-anitinoftuoran,
3-(*N*-ethyt-*N*-isoamytamino)-6-methy)-7-anitinofluoran,
3-Diethylamino-6-methyl-7-(o,p-dimethylanilino)fluoran,
3-Pyrrolidino-6-methyl-7-anilinofluoran,
3-(Cyclohexyl-*N*-methylamino)-6-methyl-7-anilinofluoran,
3-Diethylamin-7-(m-trifluoromethylanilino)fluoran,
3-N-n-dibutylamin-6-methyl-7-anilinofluoran,
3-Diethylamino-6-methyl-7-(m-methylanilino)fluoran,
3-*N*-n-dibutylamin-7-(o-chloroanilino)fluoran,
3-(*N*-ethyl-*N*-tetrahydrofurfurylamin)-6-methyl-7-anilino-fluoran,
3-(*N*-methyl-*N*-propylamin)-6-methyl-7-anilinofluoran,
3-(*N*-ethyl-*N*-ethoxypropylamin)-6-methyl-7-anilinofluoran,
3-(*N*-ethyl-*N*-isobutylamin)-6-methyl-7-anilinofluoran und/oder
3-Dipentylamin-6-methyl-7-anilinofluoran.

Die Farbbildner können als Einzelstoffe als auch als beliebige Gemische zweier oder mehrerer Farbbildner zur Anwendung kommen, vorausgesetzt die wünschenswerten anwendungstechnischen Eigenschaften der erfindungsgemäßen Aufzeichnungsmaterialien leiden darunter nicht.

Der Farbbildner liegt vorzugsweise in einer Menge von etwa 5 bis etwa 30 Gew.-%, besonders bevorzugt in einer Menge von etwa 8 bis etwa 20 Gew.-%, bezogen auf den gesamten Feststoffgehalt der wärmeempfindlichen Schicht, vor.

Zur Steuerung spezieller anwendungstechnischer Eigenschaften kann es vorteilhaft sein, wenn mindestens zwei unter die Formel (I) fallende Verbindungen als Farbentwickler in der wärmeempfindlichen Schicht vorliegen.

Desgleichen können ein oder mehrere weitere (bis)phenolische oder nicht-phenolische Farbentwickler zusätzlich zu den Verbindungen der Formel (I) in der wärmeempfindlichen farbbildenden Schicht vorliegen.

Neben dem mindestens einem Farbbildner und dem mindestens einem Farbentwickler können in der wärmeempfindlichen farbbildenden Schicht ein oder mehrere Sensibilisierungsmittel, auch thermische Lösungsmittel genannt, vorliegen, was den Vorteil hat, dass die Steuerung der thermischen Druckempfindlichkeit leichter zu realisieren ist.

Generell kommen als Sensibilisierungsmittel vorteilhafterweise kristalline Stoffe in Betracht, deren Schmelzpunkt zwischen etwa 90 und etwa 150 °C liegt und die im geschmolzenen Zustand die farbbildenden Komponenten (Farbbildner und Farbentwickler) lösen, ohne die Bildung des Farbkomplexes zu stören.

Vorzugsweise ist das Sensibilisierungsmittel ein Fettsäureamid, wie Stearamid, Beheneamid oder Palmitamid, ein Ethyien-bis-fettsäureamid, wie *N,N'*-Ethylen-bis-stearinsäureamid oder *N,N'*-Ethylen-bis-ölsäureamid, ein Fettsäurealkanolamid, wie *N*-(Hydroxymethyl)stearamid, *N*-Hydroxymethylpalmitamid oder Hydroxyethylstearamid, ein Wachs, wie Polyethylenwachs oder Montanwachs, ein Carbonsäureester, wie Dimethylterephthalat, Dibenzylterephthalat, Benzyl-4-benzyloxybenzoat, Di-(4-methylbenzyl)oxalat, Di-(4-chlorbenzyl)oxalat oder Di-(4-benzyl)oxalat, ein aromatischer Ether, wie 1,2-Diphenoxy-ethan, 1,2-Di-(3-methylphenoxy)ethan, 2-Benzyloxynaphthalin oder 1,4-Diethoxynaphthalin,

ein aromatisches Sulfon, wie Diphenylsulfon, und/oder ein aromatisches Sulfonamid, wie Benzolsulfonanilid oder *N*-Benzyl-4-toluolsulfonamid oder aromatische Kohlenwasserstoffe, wie 4-Benzylbiphenyl.

Das Sensibilisierungsmittel liegt vorzugsweise in einer Menge von etwa 10 bis etwa 40 Gew.-%, besonders bevorzugt in einer Menge von etwa 15 bis etwa 25 Gew.-%, bezogen auf den gesamten Feststoffgehalt der wärmeempfindlichen Schicht, vor.

In einer weiteren bevorzugten Ausführungsform liegt neben dem Farbbildner, dem phenolfreien Farbentwickler und dem Sensibilisierungsmittel optional mindestens ein Stabilisator (Alterungsschutzmittel) in der wärmeempfindlichen, farbbildenden Schicht vor.

Bei dem Stabilisator handelt es sich vorzugsweise um sterisch gehinderte Phenole, besonders bevorzugt um 1,1,3-Tris-(2-methyl-4-hydroxy-5-cyclohexyl-phenyl)butan, 1,1,3-Tris-(2-methyl-4-hydroxy-5-*tert*-butylphenyl)butan, 1,1-Bis-(2-methyl-4-hydroxy-5-*tert*-butyl-phenyl)butan.

Auch Harnstoff-Urethan-Verbindungen der allgemeinen Formel (II) (Handelsprodukt UU) oder vom 4,4'-Dihydroxydiphenylsulfon abgeleitete Ether, wie 4-Benzyloxy-4'-(2-methylglycidyloxy)-diphenylsulfon (Handelsname NTZ-95®, Nippon Soda Co. Ltd.), oder oligomere Ether der allgemeinen Formel (III) (Handelsname D90®, Nippon Soda Co. Ltd.) sind als Stabilisatoren im erfindungsgemäßen Aufzeichnungsmaterial einsetzbar.

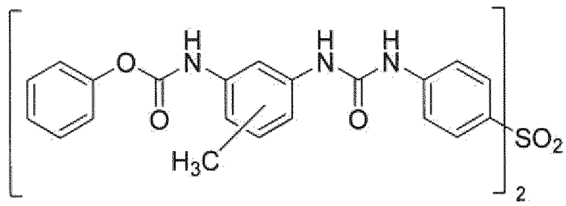

(II)

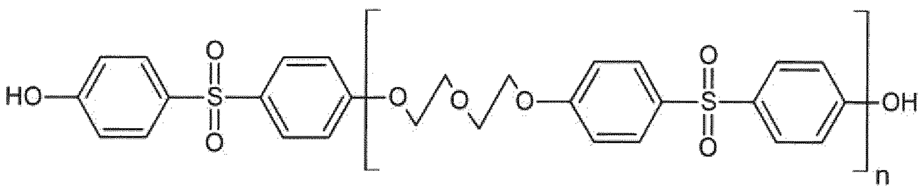

(III)

Besonders bevorzugt sind die Harnstoff-Urethan-Verbindungen der allgemeinen Formel (II).

Der Stabilisator liegt vorzugsweise in einer Menge von 0,2 bis 0,5 Gew.-Teilen, bezogen auf 1 Gew.-Teil des mindestens einen phenolfreien Farbentwicklers der Verbindung der Formel (I), vor.

In einer weiteren bevorzugten Ausführungsform liegt in der wärmeempfindlichen farbbildenden Schicht mindestens ein Bindemittel vor. Bei diesem handelt es sich vorzugsweise um wasserlösliche Stärken, Stärkederivate, stärkebasierte Biolatices vom EcoSphere®-Typ, Methylcellulose, Hydroxyethylcellulose, Carboxymethylcellulosen, partiell oder vollständig verseifte Polyvinylalkohole, chemisch modifizierte Polyvinylalkohole oder StyrolmaleinsäureanhydridCopolymere, Styrolbutadien-Copolymere, Acrylamid-(Meth)acrylat-Copolymere, Acrylamid-Acrylat-Methacrylat-Terpolymere, Polyacrylate, Poly(meth)-acrylsäureester, Acrylat-Butadien-Copolymere, Polyvinylacetate und/oder Acrylnitril-Butadien-Copolymere.

In einer weiteren bevorzugten Ausführungsform liegt mindestens ein Trennmittel (Antihaftmittel) oder Gleitmittel in der wärmeempfindlichen farbbildenden Schicht vor. Bei diesen Mitteln handelt es sich vorzugsweise um Fettsäure-Metallsalze, wie z. B. Zinkstearat oder Calciumstearat, oder auch Behenatsalze, synthetische Wachse, z. B. in Form von Fettsäureamiden, wie z. B. Stearinsäureamid und Behensäureamid, Fettsäurealkanolamide, wie z. B. Stearinsäuremethylolamid, Paraffinwachse verschiedener Schmelzpunkte, Esterwachse unterschiedlicher Molekulargewichte, Ethylenwachse, Propylenwachse unterschiedlicher Härten und/oder natürliche Wachse, wie z. B. Carnaubawachs oder Montanwachs.

Das Trennmittel liegt vorzugsweise in einer Menge von etwa 1 bis etwa 10 Gew.-%, besonders bevorzugt in einer Menge von etwa 3 bis etwa 6 Gew.-%, bezogen auf den gesamten Feststoffgehalt der wärmeempfindlichen Schicht, vor.

In einer weiteren bevorzugten Ausführungsform enthält die wärmeempfindliche farbbildende Schicht Pigmente. Der Einsatz dieser hat unter anderem den Vorteil, dass diese auf ihrer Oberfläche die im thermischen Druckprozess entstehende Chemikalien-Schmelze fixieren können. Auch kann über Pigmente die Oberflächenweiße und Opazität der

wärmeempfindlichen farbbildenden Schicht und deren Bedruckbarkeit mit konventionellen Druckfarben gesteuert werden. Schließlich besitzen Pigmente eine "Extenderfunktion", beispielsweise für die relativ teuren farbgebenden Funktionschemikalien.

Besonders geeignete Pigmente sind anorganische Pigmente, sowohl synthetischer als auch natürlicher Herkunft, vorzugsweise Clays, gefällte oder natürliche Calciumcarbonate, Aluminiumoxide, Aluminiumhydroxide, Kieselsäuren, gefällte und pyrogene Kieselsäuren (z. B. Aerodisp®-Typen), Diathomeenerden, Magnesiumcarbonate, Talk, aber auch organische Pigmente, wie Hohlpigmente mit einer Styrol/Acrylat-Copolymer-Wand oder Harnstoff/Formaldehyd-Kondensationspolymere. Diese können alleine oder in beliebigen Mischungen verwendet werden.

Die Pigmente liegen vorzugsweise in einer Menge von etwa 20 bis etwa 50 Gew.-%, besonders bevorzugt in einer Menge von etwa 30 bis etwa 40 Gew.-%, bezogen auf den gesamten Feststoffgehalt der wärmeempfindlichen Schicht, vor.

Zum Steuern der Oberflächenweiße des erfindungsgemäßen wärmeempfindlichen Aufzeichnungsmaterials können optische Aufheller in die wärmeempfindliche farbbildende Schicht eingebaut werden. Bei diesen handelt es sich vorzugsweise um Stilbene.

Um bestimmte streichtechnische Eigenschaften zu verbessern, ist es im Einzelfall bevorzugt, zu den genannten Bestandteilen des erfindungsgemäßen wärmeempfindlichen Aufzeichnungsmaterials weitere Bestandteile, insbesondere Rheologie-Hilfsmittel, wie z. B. Verdicker und/oder Tenside, hinzuzufügen.

Das Flächenauftragsgewicht der (trockenen) wärmeempfindlichen Schicht beträgt vorzugsweise etwa 1 bis etwa 10 g/m$^2$, bevorzugt etwa 3 bis etwa 6 g/m$^2$,

In einer besonders bevorzugten Ausführungsform handelt es sich bei dem wärmeempfindlichen Aufzeichnungsmaterial um ein solches nach Anspruch 9, wobei als Farbbildner ein Farbstoff des Fluoran-Typs eingesetzt wird und zusätzlich ein Sensibilisierungsmittel, ausgewählt aus der Gruppe bestehend aus Fettsäureamiden, aromatischen Sulfonen und/oder aromatischen Ethern, vorliegt. In dieser bevorzugten Ausführungsform ist es auch vorteilhaft, dass etwa 1,5 bis etwa 4 Gew.-Teile des phenolfreien Farbentwicklers nach Anspruch 1, bezogen auf 1 Gew.-Teil Farbbildner, vorliegen.

Die im Zusammenhang mit der Verbindung der Formel (I) aufgeführten bevorzugten Ausführungsformen gelten auch für das erfindungsgemäße wärmeempfindliche Aufzeichnungsmaterial.

Das erfindungsgemäße wärmeempfindliche Aufzeichnungsmaterial lässt sich mit bekannten Herstellungsverfahren gewinnen.

Es ist jedoch bevorzugt, das erfindungsgemäße Aufzeichnungsmaterial mit einem Verfahren zu gewinnen, bei dem auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-%, bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min aufgetragen und getrocknet wird.

Dieses Verfahren ist insbesondere unter wirtschaftlichen Gesichtspunkten vorteilhaft.

Wird der Wert des Feststoffgehaltes von etwa 20 Gew.-% unterschritten, dann verschlechtert sich die Wirtschaftlichkeit, da eine große Menge von Wasser aus dem Strich durch schonende Trocknung in kurzer Zeit entfernt werden muss, was sich nachteilig auf die Streichgeschwindigkeit auswirkt. Wird auf der anderen Seite der Wert von 75 Gew.-% überschritten, dann führt dies lediglich zu einem erhöhten technischen Aufwand, um die Stabilität des Streichfarben-Vorhangs während des Beschichtungsprozesses zu gewährleisten.

Beim Curtain-Coating-Beschichtungsverfahren (Vorhangbeschichtungsverfahren) wird ein frei fallender Vorhang einer Beschichtungsdispersion gebildet. Durch freien Fall wird die in Form eines dünnen Filmes (Vorhangs) vorliegende Beschichtungsdispersion auf ein Substrat "gegossen", um die Beschichtungsdispersion auf das Substrat aufzubringen. Die DE 10196052 T1 offenbart den Einsatz des Curtain-Coating-Beschichtungsverfahrens bei der Herstellung von Informationsaufzeichnungsmaterialien u.a. auch von wärmeempfindlichen Aufzeichnungsmaterialien, wobei mehrschichtige Aufzeichnungsschichten durch Aufbringen des aus mehreren Beschichtungsdispersionsfilmen bestehenden Vorhangs auf Substrate realisiert werden (Geschwindigkeit max. 200 m/min).

Die Einstellung der Betriebsgeschwindigkeit der Streichanlage auf mindestens etwa 400 m/min hat sowohl betriebswirtschaftliche als auch technische Vorteile. Bevorzugt beträgt die Betriebsgeschwindigkeit mindestens etwa 750 m/min, besonders bevorzugt mindestens etwa 1000 m/min und ganz besonders bevorzugt mindestens etwa 1500 m/min. Es war insbesondere überraschend, dass selbst bei letztgenannter Geschwindigkeit das erhaltene wärmeempfindliche Aufzeichnungsmaterial in keiner Weise beeinträchtigt ist und die Betriebsdurchführung selbst bei dieser hohen Geschwindigkeit optimal abläuft.

In einer bevorzugten Ausführungsform des erfindungsgemäßen Verfahrens weist die wässrige entlüftete Auftragssuspension eine Viskosität von etwa 150 bis etwa 800 mPas (Brookfield, 100 U/min, 20 °C) auf. Wird der Wert von etwa 150 mPas unterschritten bzw. der Wert von etwa 800 mPas überschritten, dann führt dies zu einer mangelhaften Lauffähigkeit der Streichmasse am Streichaggregat. Besonders bevorzugt beträgt die Viskosität der wässrigen entlüfteten Auftragssuspension etwa 200 bis etwa 500 mPas.

In einer bevorzugten Ausführungsform kann zur Optimierung des Verfahrens die Oberflächenspannung der wässrigen Auftragssuspension auf etwa 25 bis etwa 60 mN/m, bevorzugt auf etwa 35 bis etwa 50 mN/m (gemessen entsprechend der statischen Ringmethode nach Du Noüy, DIN 53914), eingestellt werden.

Die Ausbildung der wärmeempfindlichen farbbildenden Schicht kann on-line oder in einem separaten Streichvorgang off-line erfolgen. Dies gilt auch für eventuell nachfolgend aufgetragene Schichten oder Zwischenschichten.

Es ist vorteilhaft, wenn die getrocknete wärmeempfindliche farbbildende Schicht einer Glätt-Maßnahme unterzogen wird. Hierbei ist es vorteilhaft, die Bekk-Glätte, gemessen nach ISO 5627:1995-03, auf etwa 100 bis etwa 1000 sec., vorzugsweise auf etwa 250 bis etwa 600 sec., einzustellen.

Die Oberflächenrauigkeit (PPS) nach ISO 8791-4:2008-05 liegt vorzugsweise im Bereich von etwa 0,50 bis etwa 2,50 μm, besonders bevorzugt im Bereich von 1,00 und 2,00 μm.

Die im Zusammenhang mit der Verbindung der Formel (I) aufgeführten bevorzugten Ausführungsformen gelten ebenfalls für das erfindungsgemäße Verfahren zur Herstellung des erfindungsgemäßen wärmeempfindlichen Aufzeichnungsmaterials.

Die vorliegende Erfindung betrifft auch ein wärmeempfindliches Aufzeichnungsmaterial, welches mit dem vorstehend geschilderten Verfahren erhältlich ist.

Das vorstehend geschilderte Verfahren ist unter wirtschaftlichen Gesichtspunkten vorteilhaft und erlaubt eine hohe Verfahrensführung der Streichanlage sogar bei einer Geschwindigkeit von mehr als 1500 m/min, ohne dass es zu Beeinträchtigungen des Verfahrenserzeugnisses, das heißt des erfindungsgemäßen wärmeempfindlichen Aufzeichnungsmaterials, kommt. Die Verfahrensführung kann on-line und off-line erfolgen, was eine wünschenswerte Flexibilität zur Folge hat.

Das erfindungsgemäße wärmeempfindliche Aufzeichnungsmaterial ist vorzugsweise phenolfrei und für POS(point-of-sale)-, Etiketten- und/oder Ticket-Anwendungen gut geeignet. Es eignet sich auch zur Herstellung von Parkscheinen, Fahrkarten, Eintrittskarten, Lotto- und Wettscheinen etc., welche im Thermodirektverfahren bedruckt werden können und eine hohe Beständigkeit der darauf aufgezeichneten Bilder beim Inkontaktbringen des Schriftbildes mit hydrophoben Stoffen, wie Weichmachern, Klebstoffen, fettigen oder öligen Stoffen, etc., benötigen.

Überraschenderweise hat es sich gezeigt, dass es möglich ist, mit den erfindungsgemäßen Farbentwicklern der Formel (I) wärmeempfindliche Aufzeichnungsmaterialien bereitzustellen, welche sich durch eine herausragende Beständigkeit des Schriftbildes gegenüber hydrophoben Agenzien auszeichnen und mit denen eine gute Qualität des Druckbildes (hohe optische Dichte (o.D.) des Druckbildes) erreicht wird.

Als Vergleichsentwickler wurden nicht-phenolische Farbentwickler des Standes der Technik herangezogen, nämlich ein Sulfonylharnstoff (Pergafast 201® (**PF201**), BASF) und ein Harnstoff-Derivat **Z** (*N*-(2-(3-Phenylureido)phenyl)benzol-sulfonamid).

Die Erfindung wird nachfolgend anhand nicht beschränkter Beispiele im Detail erläutert.

Beispiele:

Herstellung der erfindungsgemäßen Verbindungen der Formel (I).

Die Verbindungen **I-XXIV** (Tabelle 2) wurden wie folgt hergestellt:

Stufe A1 - Herstellung der Sulfonamide

Zu einer Lösung aus 20 mmol aromatischem Diamin und 20 mmol Pyridin in 125 mL Dichlormethan wird eine Lösung aus 10 mmol des entsprechenden Sulfonylchlorids in 75 mL Dichlormethan bei 0 °C unter Rühren tropfenweise zugegeben. Die Reaktionslösung wird 16 Stunden bei Raumtemperatur gerührt und anschließend mit 100 mL Wasser versetzt. Die organische Phase wird abgetrennt und mit 250 mL einer 5%igen wässrigen Natriumhydroxid-Lösung versetzt. Die wässrige Phase wird mit 100 mL Dichlormethan gewaschen und durch Zugabe 25%iger Salzsäure neutral gestellt. Nach mehrmaliger Extraktion mit 100 mL Dichlormethan werden die vereinigten organischen Phasen mit 200 mL Wasser gewaschen und über Magnesiumsulfat getrocknet. Nach Entfernen des Lösungsmittels im Vakuum bleibt das Sulfonamid als Feststoff zurück. Die Sulfonamide wurden ohne weitere Aufreinigung in den Stufen B bzw. C verwendet.

Für die Gewinnung der Vorläuferverbindungen der Produkte **IX** und **XII** genügt eine einfache Filtration nach Versetzen der Reaktionslösung mit Wasser.

Stufe A2 - Herstellung der Sulfonamide

Zu einer Lösung aus 80 mmol aromatischem Amin und 240 mmol Kaliumcarbonat in 500 mL Dichlorethan wird eine Lösung aus 80 mmol des entsprechenden Sulfonylchlorids in 150 mL Dichlorethan bei Raumtemperatur unter Rühren tropfenweise zugegeben. Das Reaktionsgemisch wird sechs Stunden refluxiert und anschließend mit 300 mL Ethylacetat und 300 mL Wasser versetzt. Die wässrige Phase wird durch Zugabe 25%iger Salzsäure sauer gestellt. Die

Phasen werden getrennt. Nach mehrmaliger Extraktion der wässrigen Phase mit 200 mL Ethylacetat werden die vereinigten organischen Phasen mit 200 mL Wasser gewaschen und über Magnesiumsulfat getrocknet. Nach Entfernen der Lösungsmittel im Vakuum bleibt das Sulfonamid als Feststoff zurück. Die Sulfonamide wurden ohne weitere Aufreinigung in der Stufe B verwendet.

Stufe A3 - Herstellung der Sulfonamide

Zu einer Lösung aus 27,5 mmol Natriumhydrid (60%ig in Öl) in 25 mL *abs.* THF wird eine Lösung aus 25,0 mmol aromatischem Amin in 35 mL *abs.* THF bei 0 °C unter Rühren und Schutzgasatmosphäre tropfenweise zugegeben. Nach zweistündigem Rühren bei Raumtemperatur wird eine Lösung aus 25,0 mmol des entsprechenden Sulfonylchlorids in 10 mL *abs.* THF bei 0 °C unter Rühren tropfenweise zugegeben. Die Reaktionslösung wird 40 Stunden bei Raumtemperatur gerührt und anschließend mit 100 mL Wasser und 100 mL Dichlormethan versetzt. Die wässrige Phase wird durch Zugabe 5%iger wässriger Natriumhydroxid-Lösung alkalisch gestellt. Die Phasen werden getrennt. Die wässrige Phase wird mit 100 mL Dichlormethan gewaschen und durch Zugabe 25%iger Salzsäure neutral gestellt. Nach mehrmaliger Extraktion mit 100 mL Dichlormethan werden die vereinigten organischen Phasen mit 200 mL Wasser gewaschen und über Magnesiumsulfat getrocknet. Nach Entfernen des Lösungsmittels im Vakuum bleibt das Sulfonamid als Feststoff zurück. Die Sulfonamide wurden ohne weitere Aufreinigung in der Stufe B verwendet.

Stufe B - Reduktion der Nitro-Gruppe zum primären Amin

Zu einer Lösung aus 8,0 mmol des Produktes aus Stufe A1/A2/A3 in 140 mL Ethylacetat werden unter Rühren 28,0 mmol (Produkte aus Stufe A1) bzw. 56,0 mmol (Produkte aus Stufe A2/A3) $SnCl_2 \cdot 2H_2O$ bei Raumtemperatur zugegeben. Die Reaktionslösung wird refluxiert. Der Reaktionsverlauf wird mittels Dünnschichtchromatographie verfolgt (Eluenten: Cyclohexan/Ethylacetat 1:1). Nach Beenden der Reaktion (ca. 2-3 h) wird mit 70 mL Ethylacetat verdünnt, mit einer 10%igen wässrigen Kaliumcarbonat-Lösung versetzt und 30 min bei Raumtemperatur gerührt. Die Sn-Verbindungen werden abfiltriert und im Filtrat wird die wässrige von der organischen Phase getrennt. Die organische Phase wird mit 100 ml (2×) einer gesättigten wässrigen Natriumchlorid-Lösung gewaschen und über Magnesiumsulfat getrocknet. Nach Entfernen des Lösungsmittels im Vakuum erfolgt die Aufreinigung durch Umkristallisieren aus Dichlormethan und wenigen Tropfen n-Hexan.

Stufe C1 - Herstellung der Sulfonylharnstoff-Verbindungen

Zu einer Lösung aus 7,0 mmol des Produktes aus Stufe A1 in Dichlormethan (20 bis 40 mL) (bei Schwerlöslichkeit zusätzlich in 10 mL Acetonitril) wird eine Lösung aus 7,0 mmol des entsprechenden Sulfonylisocyanats in 10 mL Dichlormethan bei Raumtemperatur unter Rühren tropfenweise zugegeben. Die Reaktion wird mittels Dünnschichtchromatographie verfolgt (Eluenten: Cyclohexan/Ethylacetat 1:1). Nach Beenden der Reaktion wird das ausgefallene Produkt abfiltriert, mit Dichlormethan gewaschen und im Vakuum getrocknet. In manchen Fällen wird die Reaktionslösung im Vakuum eingeengt und die Kristallisation durch Zugabe weniger Tropfen n-Hexan ausgelöst.

Stufe C2 - Herstellung der Sulfonylharnstoff-Verbindungen

Zu einer Lösung aus 4,2 mmol des Produktes aus Stufe B in DMF (16 mL) wird eine Lösung aus 8,4 mmol des entsprechenden Sulfonylisocyanats in DMF (5 bis 10 mL) unter Rühren tropfenweise zugegeben. Die Reaktion wird mittels Dünnschichtchromatographie verfolgt (Eluenten: Cyclohexan/Ethylacetat 1:1). Nach Beenden der Reaktion wird die Reaktionslösung mit 100 mL Ethylacetat verdünnt, mit 100 mL (2x) einer gesättigten wässrigen Natriumchlorid-Lösung und abschließend mit 100 mL Wasser gewaschen. Nach Entfernen des Lösungsmittels im Vakuum erfolgt die Aufreinigung durch Umkristallisieren aus Dichlormethan und wenigen Tropfen n-Hexan.

Die Verbindungen **I bis XIX** (Tabelle 2) wurden ausgehend von den entsprechenden Phenylen-Diaminen nach den allgemeinen Vorschriften der Stufen A1 und C1 hergestellt.

Die Verbindung **XX** (Tabelle 2) wurde ausgehend von 2,6-Dinitroanilin hergestellt, das zunächst in 1,2-Diamino-3-nitrobenzol (Reaktionsschema 3, V. Milata, J. Saloň, Org. Prep. Proceed. Int., 31 (3), 347 (1999)) und final nach den allgemeinen Vorschriften der Stufen A1, B und C2 in das Endprodukt überführt wurde.

Die Verbindungen **XXI bis XXIV** (Tabelle 2) wurden ausgehend von 2,4-Dinitroanilin (**XXI** und **XXII**), 4-Nitro-1,2-phenylendiamin (**XXIII**) und 2,6-Dinitroanilin (**XXIV**) nach den allgemeinen Vorschriften der Stufen A1 (**XXIII**), A2 (**XXI** und **XXII**), A3 (**XXIV**), B (**XXI-XXIV**) und C2 (**XXI-XXIV**) hergestellt.

Die Ausgangsverbindungen sind kommerziell erhältlich.

Tabelle 2: Zusammenstellung ausgewählter Verbindungen der Formel (I)

| | Y | $Ar^1$ | $Ar^2$ | m | n |
|---|---|---|---|---|---|
| **I** | 1,2-Phenylen | $C_6H_5$ | $C_6H_5$ | 1 | 1 |
| **II** | 1,2-Phenylen | $C_6H_5$ | $4\text{-}CH_3\text{-}C_6H_4$ | 1 | 1 |
| **III** | 1,2-Phenylen | $4\text{-}CH_3\text{-}C_6H_4$ | $C_6H_5$ | 1 | 1 |
| **IV** | 1,2-Phenylen | $4\text{-}CH_3\text{-}C_6H_4$ | $4\text{-}CH_3\text{-}C_6H_4$ | 1 | 1 |
| **V** | 1,2-Phenylen | 4-(*tert*-$C_4H_9$)-$C_6H_4$ | $4\text{-}CH_3\text{-}C_6H_4$ | 1 | 1 |
| **VI** | 1,2-Phenylen | 2,4,6-Tri$CH_3$-$C_6H_4$ | $4\text{-}CH_3\text{-}C_6H_4$ | 1 | 1 |
| **VII** | 1,2-Phenylen | $4\text{-}OCH_3\text{-}C_6H_4$ | $4\text{-}CH_3\text{-}C_6H_4$ | 1 | 1 |
| **VIII** | 1,2-Phenylen | $4\text{-}Cl\text{-}C_6H_4$ | $4\text{-}CH_3\text{-}C_6H_4$ | 1 | 1 |
| **IX** | 1,2-Phenylen | $4\text{-}(NH\text{-}CO\text{-}CH_3)\text{-}C_6H_4$ | $4\text{-}CH_3\text{-}C_6H_4$ | 1 | 1 |
| **X** | 1,2-Phenylen | $4\text{-}NO_2\text{-}C_6H_4$ | $4\text{-}CH_3\text{-}C_6H_4$ | 1 | 1 |
| **XI** | 1,2-Phenylen | $2\text{-}(CO_2CH_3)\text{-}C_6H_4$ | $4\text{-}CH_3C_6H_4$ | 1 | 1 |
| **XII** | 1,2-Phenylen | 2-Naph | $4\text{-}CH_3\text{-}C_6H_4$ | 1 | 1 |
| **XIII** | 1,2-Phenylen | $C_6H_5\text{-}CH_2$ | $4\text{-}CH_3\text{-}C_6H_4$ | 1 | 1 |
| **XIV** | 1,3-Phenylen* | $C_6H_5$ | $4\text{-}CH_3\text{-}C_6H_4$ | 1 | 1 |
| **XV** | 1,3-Phenylen* | $4\text{-}CH_3\text{-}C_6H_4$ | $C_6H_5$ | 1 | 1 |
| **XVI** | 1,3-Phenylen* | $4\text{-}CH_3\text{-}C_6H_4$ | $4\text{-}CH_3\text{-}C_6H_4$ | 1 | 1 |
| **XVII** | 1,4-Phenylen* | $C_6H_5$ | $4\text{-}CH_3\text{-}C_6H_4$ | 1 | 1 |
| **XVIII** | 1,4-Phenylen* | $4\text{-}CH_3\text{-}C_6H_4$ | $C_6H_5$ | 1 | 1 |
| **XIX** | 1,4-Phenylen* | $4\text{-}CH_3\text{-}C_6H_4$ | $4\text{-}CH_3\text{-}C_6H_4$ | 1 | 1 |
| **XX** | Benzol-1,2,3-triyl | $4\text{-}CH_3\text{-}C_6H_4$ | $4\text{-}CH_3\text{-}C_6H_4$ | 1 | 2 |
| **XXI** | Benzol-1,2,4-triyl | $4\text{-}CH_3\text{-}C_6H_4$ | $C_6H_5$ | 1 | 2 |
| **XXII** | Benzol-1,2,4-triyl | $4\text{-}CH_3\text{-}C_6H_4$ | $4\text{-}CH_3\text{-}C_6H_4$ | 1 | 2 |
| **XXIII** | Benzol-1,2,5-triyl | $4\text{-}CH_3\text{-}C_6H_4$ | $4\text{-}CH_3\text{-}C_6H_4$ | 1 | 2 |
| **XXIV** | Benzol-1,2,6-triyl | $4\text{-}CH_3\text{-}C_6H_4$ | $4\text{-}CH_3\text{-}C_6H_4$ | 1 | 2 |
| *: Vergleichsbeispiele | | | | | |

Analytische Daten:

**I**, $C_{19}H_{17}N_3O_5S_2$, M = 431.5, *N*-((2-(Phenylsulfonamido)phenyl)carbamoyl)benzol-sulfonamid

MS (ESI): m/z (%) = 430.0 (14) $[M\text{-}H]^-$, 273.0 (100) $[M\text{-}H\text{-}Ar^2SO_2NH_2]^-$, 247.0 (15) $[M\text{-}H\text{-}Ar^2SO_2NCO]^-$.

$^1$H-NMR (500 MHz, DMSO-$d_6$): $\delta$ (ppm) = 11.66 (1H, s), 9.60 (1H, s), 8.57 (1H, s), 8.01-7.99 (2H, m), 7.86 (1H, dd, *J* = 8.3, 1.3 Hz), 7.73-7.69 (1H, m), 7.68-7.62 (5H, m), 7.56-7.53 (2H, m), 7.16-7.12 (1H, m), 6.80 (1H, ddd, *J* = 8.9, 7.9, 1.4 Hz), 6.39 (1H, dd, *J* = 7.9, 1.1 Hz).

$^{13}$C-NMR (126 MHz, DMSQ-$d_6$): $\delta$ (ppm) =149.12 (NHCONH), 139.78, 138.87, 135.56, 133.39, 133.01, 129.10, 129.03, 127.82, 127.27, 127.23, 127.08, 125.28, 123.15, 120.75.

**II,** $C_{20}H_{19}N_3O_5S_2$, M = 445.5, *N*-((2-(Phenylsulfonamido)phenyl)carbamoyl) tosylamid

MS (ESI): m/z (%) = 444.0 (23) $[M\text{-}H]^-$, 273.0 (100) $[M\text{-}H\text{-}Ar^2SO_2NH_2]^-$, 247.1 (21) $[M\text{-}H\text{-}Ar^2SO_2NCO]^-$.
$^1$H-NMR (500 MHz, DMSO-$d_6$): $\delta$ (ppm) = 11.58 (1H, s), 9.60 (1H, s), 8.56 (1H, s), 7.89-7.86 (3H, m), 7.68-7.66

(2H, m), 7.65-7.62 (1H, m), 7.56-7.53 (2H, m), 7.44-7.43 (2H, m), 7.16-7.12 (1H, m), 6.79 (1H, ddd, *J* = 8.9, 7.7, 1.4 Hz), 6.38 (1H, dd, *J* = 7.9, 1.3 Hz), 2.39 (3H, s).
$^{13}$C-NMR (126 MHz, DMSO-$d_6$): δ (ppm) = 149.16 (NHCONH), 143.93, 138.90, 136.95, 135.67, 133.02, 129.52, 129.05, 127.84, 127.37, 127.25, 127.10, 125.22, 123.08, 120.68, 21.00 ($\underline{C}H_3$).

**III,** $C_{20}H_{19}N_3O_5S_2$, M = 445.5, *N*-(2-(3-(Phenylsulfonyl)ureido)phenyl)tosylamid

MS (ESI): m/z (%) = 444.0 (17) [M-H]$^-$, 287.0 (100) [M-H-Ar$^2$SO$_2$NH$_2$]$^-$, 261.1 (7) [M-H-Ar$^2$SO$_2$NCO]$^-$.
$^1$H-NMR (500 MHz, DMSO-$d_6$): δ (ppm) = 11.66 (1H, s), 9.51 (1H, s), 8.56 (1H, s), 8.01-7.99 (2H, m), 7.85 (1H, dd, *J* = 8.3, 1.4 Hz), 7.73-7.69 (1H, m), 7.66-7.63 (2H, m), 7.56-7.54 (2H, m), 7.35-7.33 (2H, m), 7.15-7.11 (1H, m), 6.81 (1H, ddd, 9.1, 7.7, 1.4 Hz), 6.43-6.42 (1H, m), 2.36 (3H, s).
$^{13}$C-NMR (126 MHz, DMSO-$d_6$): δ (ppm) = 149.13 (NHCONH), 143.34, 139.80, 136.07, 135.48, 133.38, 129.47, 129.09, 127.70, 127.26, 127.18, 127.13, 125.43, 123.16, 120.73, 20.96 ($CH_3$).

**IV,** $C_{21}H_{21}N_3O_5S_2$, M = 459.5, *N*-((2-(Tosylamido)phenyl)carbamoyl)tosyiamid

MS (ESI): m/z (%) = 458.1 (26) [M-H]$^-$, 287.0 (100) [M-H-Ar$^2$SO$_2$NH$_2$]$^-$, 261.0 (10) (M-H-Ar$^2$SO$_2$NCO]$^-$.
$^1$H-NMR (500 MHz, DMSO-$d_6$): δ (ppm) = 11.58 (1H, s), 9.50 (1H, s), 8.55 (1H, s), 7.89-7.85 (3H, m), 7.56-7.54 (2H, m), 7.44-7.42 (2H, m), 7.35-7.33 (2H, m), 7.15-7.11 (1H, m), 6.80 (1H, ddd, *J* = 9.0, 7.7, 1.4 Hz), 6.43-6.41 (1H, m), 2.39 (3H, s), 2.36 (3H, s).
$^{13}$C-NMR (126 MHz, DMSO-$d_6$): δ (ppm) = 149.15 (NHCONH), 143.88, 143.33, 136.98, 136.11, 135.59, 129.49, 129.45, 127.70, 127.36, 127.19, 127.15, 125.37, 123.07, 120.65, 20.98 ($\underline{C}H_3$), 20.94 ($CH_3$).

**V,** $C_{24}H_{27}N_3O_5S_2$, M = 501.6, *N*-(2-(3-Tosylureido)phenyl)-4-*tert*-butylbenzol-sulfonamid

MS (ESI): m/z (%) = 500.1 (56) [M-H]$^-$, 329.1 (100) [M-H-Ar$^2$SO$_2$NH$_2$)$^-$.

$^1$H-NMR (500 MHz, DMSO-$d_6$): δ (ppm) = 11.57 (1H, s), 9.53 (1H, s), 8.55 (1H, s), 7.89-7.87 (2H, m), 7.86-7.85 (1H, m), 7.62-7.60 (2H, m), 7.58-7.56 (2H, m), 7.44-7.42 (2H, m), 7.14-7.11 (1H, m), 6.80-6.77 (1H, m), 6.42-6.40 (1H, m), 2.39 (3H, s), 1.28 (9H, s).

$^{13}$C-NMR (126 MHz, DMSO-$d_6$): δ (ppm) = 156.10, 149.16 (NH$\underline{C}$ONH), 143.89, 136.96, 136.15, 135.48, 129.51, 127.66, 127.35, 127.03, 127.03, 125.84, 125.44, 123.05, 120.68, 34.83 ($\underline{C}(CH_3)_3$), 30.71 ($C(\underline{C}H_3)_3$), 21.00 ($CH_3$).

**VI,** $C_{23}H_{25}N_3O_5S_2$, M = 487.6, *N*-(2-(3-Tosylureido)phenyl)-2,4,6-trimethyfbenzol-sulfonamid

MS (ESI): m/z (%) = 486.1 (37) [M-H]$^-$, 315.1 (100) [M-H-Ar$^2$SO$_2$NH$_2$]$^-$, 289.1 (18) [M-H-Ar$^2$SO$_2$NCO]$^-$.

$^1$H-NMR (500 MHz, DMSO-$d_6$): δ (ppm) = 11.66 (1H, s), 9.26 (1H, s), 8.62 (1H, s), 7.89-7.87 (3H, m), 7.44-7.42 (2H, m), 7.18-7.14 (1H, m), 6.98 (2H, s), 6.77 (1H, ddd, *J* = 9.0, 7.7, 1.4 Hz), 6.26 (1H, dd, *J* = 7.9, 1.3 Hz), 2.39 (3H, s), 2.29 (6H, s), 2.24 (3H, s).

$^{13}$C-NMR (126 MHz, DMSO-$d_6$): δ (ppm) = 149.26 (NHCONH), 143.87, 142.02, 138.89, 136.96, 136,06, 133.30, 131.54, 129.48, 127.99, 127.83, 127.35, 124.76, 123.09, 120.55, 22.42 (2×$\underline{C}H_3$), 20.98 ($\underline{C}H_3$), 20.36 ($CH_3$).

**VII,** $C_{21}H_{21}N_3O_6S_2$, M = 475.5, *N*-(2-(3-Tosylureido)phenyl)-4-methoxybenzol-sulfonamid

MS (ESI): m/z (%) = 474.1 (28) [M-H]$^-$, 303.0 (100) [M-H-Ar$^2$SO$_2$NH$_2$]$^-$.

$^1$H-NMR (500 MHz, DMSO-$d_6$): δ (ppm) = 11.59 (1H, s), 9.42 (1H, s), 8.56 (1H, s), 7.89-7.85 (3H, m), 7.60-7.58 (2H, m), 7.44-7.42 (2H, m), 7.15-7.12 (1H, m), 7.07-7.05 (2H, m), 6.82 (1H, ddd, *J* = 8.7, 7.7, 1.0 Hz), 6.43 (1H, dd, *J* = 7.9, 1.1 Hz), 3.82 (3H, s), 2.39 (3H, s).

$^{13}$C-NMR (126 MHz, DMSO-$d_6$): δ (ppm) = 162.53, 149.16 (NHCONH), 143.90, 136.96, 135.64, 130.47, 129.50, 129.34, 127.70, 127.34, 127.30, 125.46, 123.07, 120.58, 114.17, 55.60 ($O\underline{C}H_3$), 20.99 ($CH_3$).

**VIII,** $C_{20}H_{18}ClN_3O_5S_2$, M = 480.0, *N*-(2-(3-Tosylureido)phenyl)-4-chlorbenzol-sulfonamid

MS (ESI): m/z (%) = 478.0 (29) $[M-H]^-$, 307.0 (100) $[M-H-Ar^2SO_2NH_2]^-$ 281.0 (49) $[M-H-Ar^2SO_2NCO]^-$.

$^1H$-NMR (500 MHz, DMSO-$d_6$): δ (ppm) = 11.56 (1H, s), 9.70 (1H, s), 8.55 (1H, s), 7.88-7.86 (3H, m), 7.67-7.65 (2H, m), 7.63-7.61 (2H, m), 7.44-7.43 (2H, m), 7.18-7.14 (1H, m), 6.84 (1H, ddd, *J* = 9.2, 7.9, 1.4 Hz), 6.44 (1H, dd, *J* = 7.9, 1.5 Hz), 2.39 (3H, s).

$^{13}C$-NMR (126 MHz, DMSO-$d_6$): δ (ppm) = 149.13 (NHCONH), 143.93, 137.95, 137.77, 136.91, 135.65, 129.52, 129.24, 129.04, 128.00, 127.35, 127.28, 124.97, 123.26, 120.79, 21.00 ($CH_3$).

**IX,** $C_{22}H_{22}N_4O_6S_2$, M = 502.6, *N*-(4-(*N*-(2-(3-Tosylureido)phenyl)sulfamoyl)phenyl) acetamid

MS (ESI): m/z (%) = 501.0 (32) $[M-H]^-$, 330.1 (100) $[M-H-Ar^2SO_2NH_2]^-$.

$^1H$-NMR (500 MHz, DMSO-$d_6$): δ (ppm) = 11.60 (1H, s), 10.30 (1H, s), 9.45 (1H, s), 8.56 (1H, s), 7.89-7.86 (3H, m), 7.75-7.73 (2H, m), 7.60-7.57 (2H, m), 7.44-7.42 (2H, m), 7.15-7.12 (1H, m), 6.81 (1H, ddd, *J* = 9.0, 7.6, 1.4 Hz), 6.40 (1H, dd, *J* = 7.9, 1.4 Hz), 2.39 (3H, s), 2.09 (3H, s).

$^{13}C$-NMR (126 MHz, DMSO-$d_6$): δ (ppm) = 169.01 ($NHCOCH_3$), 149.19 (NHCONH), 143.91, 143.26, 136.96, 135.69, 132.36, 129.51, 128.38, 127.73, 127.35, 127.28, 125.41, 123.08, 120.59, 118.34, 24.11 ($CH_3$), 21.00 ($CH_3$).

**X,** $C_{20}H_{18}N_4O_7S_2$, M = 490.5, *N*-((2-(4-Nitrophenylsulfonamido)phenyl)carbamoyl) tosylamid

MS (ESI): m/z (%) = 489.0 (31) $[M-H]^-$, 318.0 (55) $[M-H-Ar^2SO_2NH_2]^-$, 292.0 (100) $[M-H-Ar^2SO_2NCO]^-$.
$^1H$-NMR (500 MHz, DMSO-$d_6$): δ (ppm) = 11.51 (1H, s), 9.97 (1H, s), 8.52 (1H, s), 8.40-8.37 (2H, m), 7.92-7.89 (2H, m), 7.87-7.84 (3H, m), 7.44-7.42 (2H, m), 7.20-7.16 (1H, m), 6.85 (1H, ddd, *J* = 8.9, 7.7, 1.4 Hz), 6.48 (1H, dd, *J* = 7.9, 1.3 Hz), 2.39 (3H,s).
$^{13}C$-NMR (126 MHz, DMSO-$d_6$): δ (ppm) = 149.88, 149.13 (NHCONH), 144.49, 143.96, 136.89, 135.60, 129.52, 128.67, 128.24, 127.50, 127.33, 124.71, 124.47, 123.50, 121.08, 21.00 ($CH_3$).

**XI,** $C_{22}H_{21}N_3O_7S_2$, M = 503.5, Methyl 2-(*N*-(2-(3-tosylureido)phenyl)sulfamoyl) benzoate

MS (ESI): m/z (%) = 502.1 (32) $[M-H]^-$, 331.0 (100) $[M-H-Ar^2SO_2NH_2]^-$, 305.0 (31) $[M-H-Ar^2SO_2NCO]^-$.

$^1H$-NMR (500 MHz, DMSO-$d_6$): δ (ppm) = 11.53 (1H, s), 9.21 (1H, s), 8.53 (1H, s), 7.88-7.86 (2H, m), 7.82-7.81 (1H, m), 7.73-7.72 (2H, m), 7.67-7.65 (2H, m), 7.44-7.42 (2H, m), 7.18-7.15 (1H, m), 6.85-6.82 (1H, m), 6.55-6.54 (1H, m), 3.72 (3H, s), 2.39 (3H, s).

$^{13}C$-NMR (126 MHz, DMSO-$d_6$): δ (ppm) = 167.35 ($COOCH_3$), 149.29 (NHCONH), 143.90, 136.93, 136.57, 135.40, 133.17, 132.03, 130.94, 129.49, 129.13, 129.11, 127.91, 127.42, 127.34, 125.39, 123.43, 121.21, 52.87 ($COOCH_3$), 20.98 ($CH_3$).

**XII,** $C_{24}H_{21}N_3O_5S_2$, M = 495.6, *N*-(2-(3-Tosylureido)phenyl)naphthafin-2-sulfonamid

MS (ESI): m/z (%) = 494.1 (24) $[M-H]^-$, 323.0 (100) $[M-H-Ar^2SO_2NH_2]^-$, 297.0 (26) $[M-H-Ar^2SO_2NCO]^-$.

$^1H$-NMR (500 MHz, DMSO-$d_6$): δ (ppm) = 11.64 (1H, s), 9.73 (1H, s), 8.64 (1H, s), 8.27 (1H, d, *J* = 1.4 Hz), 8.13-8.12 (1H, m), 8.08-8.06 (1H, m), 8.04-8.02 (1H, m), 7.90-7.89 (2H, m), 7.89 (1H, dd, *J* = 8.3, 1.3 Hz), 7.80 (1H, dd, *J* = 8.7, 1.9 Hz), 7.71-7.68 (1H, m), 7.65-7.61 (1H, m), 7.45-7.43 (2H, m), 7.12-7.09 (1H, m), 6.71-6.68 (1H, m), 6.37-6.36 (1H, m), 2.39 (3H, s).

$^{13}C$-NMR (126 MHz, DMSO-$d_6$): δ (ppm) = 149.19 (NHCONH), 143.92, 136.96, 135.95, 135.74, 134.29, 131.43, 129.52, 129.26, 129.19, 128.94, 128.34, 127.84, 127.81, 127.61, 127.37, 127.22, 125.21, 123.09, 122.45, 120.67, 20.99 ($CH_3$).

**XIII,** $C_{21}H_{21}N_3O_5S_2$, M = 459.5, *N*-((2-(Benzylsutfonamido)phenyl)carbamoyl) tosylamid

MS (ESI): m/z (%) = 458.0 (16) $[M-H]^-$, 287.0 (100) $[M-H-Ar^2SO_2NH_2]^-$, 261.1 (13) $[M-H-Ar^2SO_2NCO]^-$.

$^1H$-NMR (500 MHz, DMSO-$d_6$): δ (ppm) = 11.47 (1H, s), 9.25 (1H, s), 8.40 (1H, s), 7.86-7.84 (2H, m), 7.81 (1H, dd, *J* = 8.4, 1.4 Hz), 7.39-7.34 (8H, m), 7.25-7.21 (1H, m), 7.10 (1H, ddd, *J* = 9.0, 7.6, 1.5 Hz), 4.40 (2H, s), 2.36 (3H, s).

$^{13}C$-NMR (126 MHz, DMSO-$d_6$): δ (ppm) = 149.33 (NHC̲ONH), 143.87, 136.93, 134.39, 130.83, 129.46, 129.06, 128.33, 128.16, 127.52, 127.33, 127.33, 126.50, 123.97, 121.66, 57.25 ($\underline{C}H_2$), 20.98 ($\underline{C}H_3$).

**XIV,** $C_{26}H_{19}N_3O_5S_2$, M = 445.5, *N*-((3-(Pheny)sulfonamido)phenyl)carbamoyl) tosylamid

MS (ESI): m/z (%) = 444.0 (100) $[M-H]^-$, 272.9 (7) $[M-H-Ar^2SO_2NH_2]^-$.

$^1H$-NMR (500 MHz, DMSO-$d_6$): δ (ppm) = 10.55 (1H, s), 10.24 (1H, s), 8.83 (1H, s), 7.86-7.84 (2H, m), 7.76-7.74 (2H, m), 7.59-7.56 (1H, m), 7.52-7.49 (2H, m), 7.43-7.41 (2H, m), 7.24-7.23 (1H, m), 7.10-7.07 (1H, m), 6.97 (1H, ddd, *J* = 8.2, 2.0, 0.8 Hz), 6.76 (1H, ddd, *J* = 8.1, 2.1, 0.9 Hz), 2.39 (3H, s).

$^{13}C$-NMR (126 MHz, DMSO-$d_6$): δ (ppm) = 149.05 (NHC̲ONH), 143.80, 139.46, 138.70, 138.19, 137.01, 132.79, 129.39, 129.39, 129.11, 127.45, 126.58, 114.42, 114.34, 110.23, 20.98 ($\underline{C}H_3$).

**XV,** $C_{20}H_{19}N_3O_5S_2$, M = 445.5, *N*-(3-(3-(Phenylsulfonyl)ureido)phenyl)tosylamid

MS (ESI): m/z (%) = 444.0 (100) $[M-H]^-$, 287.0 (6) $[M-H-Ar^2SO_2NH_2]^-$.

$^1H$-NMR (500 MHz, DMSO-$d_6$): δ (ppm) = 10.44 (1H, s), 10.17 (1H, s), 8.87 (1H, s), 7.99-7.97 (2H, m), 7.71-7.68 (1H, m), 7.64-7.61 (4H, m), 7.30-7.28 (2H, m), 7.24-7.23 (1H, m), 7.10-7.07 (1H, m), 6.97 (1H, ddd, *J* = 8.2, 2.1, 0.9 Hz), 6.76 (1H, ddd, *J* = 8.1, 2.1, 0.9 Hz), 2.30 (3H, s).

$^{13}C$-NMR (126 MHz, DMSO-$d_6$): δ (ppm) = 149.03 (NHCONH), 143.16, 139.90, 138.64, 138.34, 136.61, 133.28, 129.54, 129.36, 128.97, 127.37, 126.66, 114.27, 114.22, 110.04, 20.87 ($\underline{C}H_3$).

**XVI,** $C_{21}H_{21}N_3O_5S_2$, M = 459.5, *N*-((3-(Tosylamido)phenyl)carbamoyl)tosylamid

MS (ESI): m/z (%) = 458.1 (100) $[M-H]^-$, 287.0 (4) $[M-H-Ar^2SO_2NH_2]^-$.

$^1H$-NMR (500 MHz, DMSO-$d_6$): δ (ppm) = 10.53 (1H, s), 10.17 (1H, s), 8,82 (1H, s), 7.86-7.85 (2H, m), 7.65-7.63 (2H, m), 7.42-7.41 (2H, m), 7.30-7.28 (2H, m), 7.24-7.24 (1H, m), 7.10-7.06 (1H, m), 6.97-6.95 (1H, m), 6.77-6.75 (1H, m), 2.38 (3H, s), 2.30 (3H, s).

$^{13}C$-NMR (126 MHz, DMSO-$d_6$): δ (ppm) = 149.05 (NHCONH), 143.80, 143.15, 138.68, 138.35, 137.03, 136.62, 129.54, 129.39, 129.36, 127.46, 126.67, 114.22, 114.17, 109.98, 20.99 ($\underline{C}H_3$), 20.87 ($\underline{C}H_3$).

**XVII,** $C_{20}H_{19}N_3O_5S_2$, M = 445.5, *N*-((4-(Phenylsulfonamido)phenyl)carbamoyl) tosylamid

MS (ESI): m/z (%) = 444.0 (100) $[M-H]^-$, 273.0 (5) $[M-H-Ar^2SO_2NH_2]^-$.

$^1H$-NMR (500 MHz, DMSO-$d_6$): δ (ppm) = 10.47 (1H, s), 10.05 (1H, s), 8.70 (1H, s), 7.84-7.82 (2H, m), 7.70-7.68 (2H, m), 7.58-7.55 (1H, m), 7.52-7.49 (2H, m), 7.40-7.39 (2H, m), 7.20-7.18 (2H, m), 6.99-6.97 (2H, m), 2.37 (3H, s).

$^{13}C$-NMR (126 MHz, DMSO-$d_6$): δ (ppm) = 149.19 (NHCONH), 143.77, 139.39, 137.06, 134.61, 132.72, 132.72, 129.41, 129.09, 127.41, 126.59, 121.63, 119.83, 20.99 ($\underline{C}H_3$).

**XVIII,** $C_{20}H_{19}N_3O_5S_2$, M = 445.5, *N*-(4-(3-(Phenylsulfonyl)ureido)phenyl)tosylamid

MS (ESI): m/z (%) = 444.0 (100) $[M-H]^-$, 287.0 (5) $[M-H-Ar^2SO_2NH_2]^-$.

$^1H$-NMR (500 MHz, DMSO-$d_6$): δ (ppm) = 10.70 (1H, s), 9.98 (1H, s), 8.73 (1H, s), 7.96-7.95 (2H, m), 7.69-7.66 (1H, m), 7.62-7.57 (4H, m), 7.30-7.29 (2H, m), 7.20-7.18 (2H, m), 7.00-6.97 (2H, m), 2.30 (3H, s).

$^{13}$C-NMR (126 MHz, DMSO-$d_6$): δ (ppm) = 149.16 (NHCONH), 143.04, 139.94, 136.56, 134.40, 133.25, 132.96, 129.51, 128.98, 127.31, 126.64, 121.41, 119.91, 20.87 ($CH_3$).

**XIX,** $C_{21}H_{21}N_3O_5S_2$, M = 459.5, *N*-((4-(Tosylamido)phenyl)carbamoyl)tosylamid

MS (ESI): m/z (%) = 458.1 (100) $[M\text{-}H]^-$, 287.1 (4) $[M\text{-}H\text{-}Ar^2SO_2NH_2]^-$.

$^1$H-NMR (500 MHz, DMSO-$d_6$): δ (ppm) = 10.55 (1H, s), 9.97 (1H, s), 8.68 (1H, s), 7.84-7.82 (2H, m), 7.59-7.57 (2H, m), 7.40-7.39 (2H, m), 7.30-7.28 (2H, m), 7.20-7.17 (2H, m), 7.00-6.97 (2H, m), 2.37 (3H, s), 2.30 (3H, s).

$^{13}$C-NMR (126 MHz, DMSO-$d_6$): δ (ppm) = 149.17 (NHCONH), 143.75, 143.03, 137.07, 136.56, 134.44, 132.91, 129.51, 129.39, 127.39, 126.63, 121.42, 119.84, 20.97 ($CH_3$), 20.86 ($CH_3$).

**XX,** $C_{29}H_{29}N_5O_8S_3$, M = 671.8, *N*,*N*-(((3-Tosylamido-1,2-phenylen) bis(azandiyl))bis(carbonyl))bis(tosylamid)

MS (ESI): m/z (%) = 670.0 (21) $[M\text{-}H]^-$, 499.0 (100) $[M\text{-}H\text{-}Ar^2SO_2NH_2]^-$, 302.0 (70) $[M\text{-}H\text{-}Ar^2SO_2NCO\text{-}Ar^2SO_2NH_2]^-$.

$^1$H-NMR (500 MHz, DMSO-$d_6$): δ (ppm) = 11.38 (1H, s), 11.18 (1H, s), 9.47 (1H, s), 8.16 (1H, s), 7.91 (1H, s), 7.91-7.89 (2H, m), 7.80-7.78 (2H, m), 7.54-7.53 (1H, m), 7.49-7.47 (2H, m), 7.42-7.40 (4H, m), 7.29-7.28 (2H, m), 7.00-6.97 (1H, m), 6.42-6.40 (1H, m), 2.39 (3H, s), 2.38 (3H, s), 2.34 (3H, s).

$^{13}$C-NMR (126 MHz, DMSO-$d_6$): δ (ppm) = 150.34 (NHCONH), 149.01 (NHCONH), 143.91, 143.64, 143.34, 137.20, 136.81, 136.01, 135.71, 133.30, 129.52, 129.50, 129.45, 127.22, 127.20, 126.89, 125.57, 122.98, 119.81, 119.08, 21.01 ($CH_3$), 21.01 ($CH_3$), 20.95 ($CH_3$).

**XXI,** $C_{27}H_{25}N_5O_8S_3$, M = 643.7, *N*,*N*-(((4-Tosylamido-1,3-phenylen) bis(azandiyl))bis(carbonyl))bis(benzolsulfonamid)

MS (ESI): m/z (%) = 644.0 (79) $[M+H]^+$.

$^1$H-NMR (500 MHz, DMSO-$d_6$): δ (ppm) = 11.69 (1H, s), 10.62 (1H, s), 9.36 (1H, s), 8.94 (1H, s), 8.53 (1H, s), 8.00-7.98 (2H, m), 7.95-7.93 (2H, m), 7.91 (1H, d, *J* = 2.5 Hz), 7.74-7.59 (6H, m), 7.52-7.50 (2H, m), 7.33-7.32 (2H, m), 6.86 (1H, dd, *J* = 8.7, 2.5 Hz), 6.23 (1H, d, *J* = 8.7 Hz), 2.35 (3H, s).

$^{13}$C-NMR (126 MHz, DMSO-$d_6$): δ (ppm) = 149.05 (NHCONH), 148.98 (NHCONH), 143.35, 139.85, 139.72, 137.58, 136.32, 135.91, 133.45, 133.30, 129.47, 129.13, 129.01, 127.95, 127.35, 127.21, 125.84, 119.89, 113.18, 110.52, 20.98 ($CH_3$).

**XXII,** $C_{29}H_{29}N_5O_8S_3$, M = 671.8, *N*,*N*-(((4-Tosylamido-1,3-phenylen) bis(azandiyl))bis(carbonyl))bis(tosylamid)

MS (ESI): m/z (%) = 670.0 (15) $[M\text{-}H]^-$, 499.0 (100) $[M\text{-}H\text{-}Ar^2SO_2NH_2]^-$, 473.0 (2) $[M\text{-}H\text{-}Ar^2SO_2NCO]^-$, 328.0 (5) $[M\text{-}H\text{-}2\times Ar^2SO_2NH_2]^-$.
$^1$H-NMR (500 MHz, DMSO-$d_6$): δ (ppm) = 11.60 (1H, s), 10.69 (1H, s), 9.34 (1H, s), 9.18 (1H, s), 8.55 (1H, s), 7.91 (1H, d, *J* = 2.4 Hz), 7.87-7.85 (2H, m), 7.82-7.80 (2H, m), 7.51-7.50 (2H, m), 7.45-7.43 (2H, m), 7.40-7.39 (2H, m), 7.32-7.31 (2H, m), 6.82 (1H, dd, *J* = 8.7, 2.4 Hz), 6.24 (1H, d, *J* = 8.7 Hz), 2.40 (3H, s), 2.38 (3H, s), 2.35 (3H, s).
$^{13}$C-NMR (126 MHz, DMSO-$d_6$): δ (ppm) = 149.09 (NHCONH), 148.93 (NHCONH), 143.95, 143.76, 143.31, 137.61, 136.99, 136.87, 136.29, 135.91, 129.54, 129.42, 129.42, 127.92, 127.40, 127.38, 127.21, 119.77, 112.96, 110.27, 21.01 ($CH_3$), 21.00 ($CH_3$), 20.99 ($CH_3$).

**XXIII,** $C_{29}H_{29}N_5O_8S_3$, M = 671.8, *N*,*N*-(((2-Tosylamido-1,4-phenylen) bis(azandiyl))bis(carbonyl))bis(tosylamid)

MS (ESI): m/z (%) = 670.0 (60) $[M\text{-}H]^-$, 499.0 (100) $[M\text{-}H\text{-}Ar^2SO_2NH_2]^-$.
$^1$H-NMR (500 MHz, DMSO-$d_6$): δ (ppm) = 11.38 (1H, s), 10.47 (1H, s), 9.47 (1H, s), 8.68 (1H, s), 8.33 (1H, s), 7.86-7.84 (2H, m), 7.82-7.81 (2H, m), 7.62 (1H, d, *J* = 9.0 Hz), 7.54-7,52 (2H, m), 7.43-7.41 (4H, m), 7.28-7.26 (2H, m), 7.05 (1H, dd, *J* = 9.0, 2.5 Hz), 6.76 (1H, d, *J* = 2.5 Hz), 2.39 (3H, s), 2.38 (3H, s), 2.32 (3H, s).
$^{13}$C NMR (126 MHz, DMSO-$d_6$): δ (ppm) = 149.20 (NHCONH), 148.86 (NHCONH), 143.87, 143.78, 143.32,

137.05, 137.02, 135.99, 133.65, 129.88, 129.51, 129.45, 129.37, 127.43, 127.35, 127.02, 126.39, 121.75, 117.81, 117.28, 21.01 ($\underline{C}H_3$), 21.01 ($\underline{C}H_3$), 20.97 ($\underline{C}H_3$).

**XXIV,** $C_{29}H_{29}N_5O_8S_3$, M = 671.8, *N,N*-(((2-Tosylamido-1,3-phenylen) bis(azandiyl))bis(carbonyl))bis(tosylamid)

MS (ESI): m/z (%) = 670.1 (100) $[M-H]^-$, 499,0 (33) $[M-H-Ar^2SO_2NH_2]^-$, 473.0 (29) $[M-H-Ar^2SO_2NCO]^-$, 328.1 (23) $[M-H-2\times Ar^2SO_2NH_2]^-$, 302.0 (21) $[M-H-Ar^2SO_2NCO-Ar^2SO_2NH_2]^-$.
$^1$H-NMR (500 MHz, DMSO-*d*$_6$): $\delta$ (ppm) = 11.22 (2H, s), 8.91 (1H, s), 8.13 (2H, s), 7.84-7.82 (4H, m), 7.43-7.42 (4H, m), 7.40-7.38 (2H, m), 7.37-7.35 (2H, m), 7.19-7.17 (2H, m), 7.13-7.09 (1H, m), 2.39 (6H, s), 2.27 (3H, s).
$^{13}$C NMR (126 MHz, DMSO-*d*$_6$): $\delta$ (ppm) = 148.89 (NH$\underline{C}$ONH), 143.85, 143.78, 136.95, 136.62, 135.51, 129.44, 129.22, 128.40, 127.28, 126.87, 116.40, 115.44, 20.97 ($\underline{C}H_3$), 20.95 ($\underline{C}H_3$), 20.95 ($\underline{C}H_3$).

Der Auftrag einer wässrigen Auftragssuspension zur Ausbildung der wärmeempfindlichen farbbildenden Schicht eines wärmeempfindlichen Aufzeichnungspapiers erfolgte im Labormaßstabe mittels einer Stabrakel auf eine Seite eines synthetischen Basispapieres (Yupo® FP680) von 63 g/m$^2$, Nach Trocknung wurde ein thermisches Aufzeichnungsblatt erhalten. Die Auftragsmenge der wärmeempfindlichen farbbildenden Schicht lag zwischen 3,8 und 4,2 g/m$^2$.
Anhand der vorstehend gemachten Angaben wurde ein wärmeempfindliches Aufzeichnungsmaterial bzw. Thermopapier hergestellt, wobei die folgenden Rezepturen wässriger Auftragssuspensionen zur Ausbildung eines Verbundgebildes auf einem Trägersubstrat herangezogen und anschließend in üblicher Weise die weiteren Schichten, insbesondere eine Schutzschicht, ausgebildet wurden, worauf hier nicht gesondert eingegangen werden soll.
Herstellen der Dispersionen (jeweils für 1 Gew.-Teil) für die Auftragssuspensionen
Die wässrige **Dispersion A** (Farbbildnerdispersion) wird durch Mahlen von 20 Gew.-Teilen 3-*N*-n-Dibutylamin-6-methyl-7-anilinofluoran (ODB-2) mit 33 Gew.-Teilen einer 15%igen wässrigen Lösung von Ghosenex™ L-3266 (sulfonierter Polyvinylalkohol, Nippon Ghosei) in einer Perlen-Mühle hergestellt.
Die wässrige **Dispersion B** (Farbentwicklerdispersion) wird durch Mahlen von 40 Gew.-Teilen des Farbentwicklers zusammen mit 66 Gew.-Teilen einer 15%igen wässrigen Lösung von Ghosenex™ L-3266 in der Perlen-Mühle hergestellt.
Die wässrige **Dispersion C** (Sensibilisierungsdispersion) wird durch Mahlen von 40 Gew.-Teilen Sensibilisierungsmittel mit 33 Gew.-Teilen einer 15%igen wässrigen Lösung von Ghosenex™ L-3266 in einer Perlen-Mühle hergestellt,
**[0105]** Alle durch Mahlen erzeugten Dispersionen haben eine mittlere Körngröße $D_{(4,3)}$ von 0,80 bis 1,20 $\mu$m. Die Messung der Korngrößenverteilung der Dispersionen erfolgte durch Laserbeugung mit einem Coulter LS230-Gerät der Fa. Beckman Coulter.
Die **Dispersion D** (Gleitmitteldispersion) ist eine 20%ige Zinkstearat-Dispersion, bestehend aus 9 Gew.-Teilen Zn-Stearat, 1 Gew.-Teil Ghosenex™ L-3266 und 40 Gew.-Teilen Wasser.
Pigment **P** ist eine 72%ige Streichkaolin-Suspension (Lustra® S, BASF).
Der **Binder** besteht aus einer 10%igen wässrigen Polyvinylalkohollösung (Mowiol 28-99, Kuraray Europe).
Die wärmeempfindliche Auftragssuspension wird durch Mischen unter Rühren von 1 Teil **A,** 1 Teil **B,** 1 Teil **C,** 56 Teilen **D,** 146 Teilen Pigment **P** und 138 Teilen **Binder**-Lösung (alles Gew.-Teile) unter Berücksichtigung der Eintragsreihenfolge **B, D, C, P, A, Binder** hergestellt und mit Wasser auf einen Feststoffgehalt von etwa 25% gebracht.
Die so erhaltenen wärmeempfindlichen Beschichtungssuspensionen wurden herangezogen, um Verbundgebilde aus Papierträger und Thermoreaktionsschicht herzustellen.
Die thermischen Aufzeichnungsmaterialien wurden wie nachstehend beschrieben ausgewertet (siehe Tabellen 3, 4 und 5).

(1) Dynamische Farbdichte:
Die Papiere (6 cm breite Streifen) wurden thermisch unter Verwendung des Atlantek 200 Testdruckers (Fa. Atlantek, USA) mit einer Kyocera-Druckleiste von 200 dpi und 560 Ohm bei einer angelegten Spannung von 20,6 V und einer maximalen Pulsbreite von 0,8 ms mit einem Schachbrett-Muster mit 10 Energieabstufungen bedruckt. Die Bilddichte (optische Dichte, o.D.) wurde mit einem SpectroEye-Densitometer von X-Rite bei einer Energiestufe von 0,25 und 0,45 mJ/dot gemessen. Die Messunsicherheit der o.D.-Werte wird mit $\leq$2% veranschlagt.
(2) Statische Farbdichte (Starttemperatur):

Das Aufzeichnungsblatt wurde gegen eine Reihe auf unterschiedliche Temperaturen erhitzter und thermostatierter metallischer Stempel mit einem Anpressdruck von 0,2 kg/cm$^2$ und einer Kontaktzeit von 5 Sek. gepresst (Thermoprüfer TP 3000QM, Maschinenfabrik Hans Rychiger AG, Steffisburg, Schweiz). Die Bilddichte (opt. Dichte) der so erzeugten Bilder wurde mit einem SpectroEye-Densitometer von X-Rite gemessen.

Der statische Startpunkt ist definitionsmäßig die niedrigste Temperatur, bei welcher eine optische Dichte von 0,2 erreicht wird. Die Genauigkeit des Messverfahrens ist ≤±0.5 °C.

(3) Beständigkeitsprüfung des Druckbildes:

a) Weichmacherbeständigkeit:
Auf die gemäß dem Verfahren von (1) dynamisch aufgezeichnete Probe des thermischen Aufzeichnungspapiers wurde eine weichmacherhaltige Frischhaltefolie (PVC-Folie mit 20 bis 25% Dioctyladipat) unter Vermeiden von Falten und Lufteinschlüssen in Kontakt gebracht, zu einer Rolle gewickelt und 16 Stdn. gelagert. Eine Probe wurde bei Raumtemperatur (20 bis 22 °C), eine zweite bei 40 °C gelagert. Nach Abziehen der Folie wurde die Bilddichte (o.D.) gemessen und entsprechend der Formel (Gl. 1) in Bezug zu den entsprechenden Bilddichtewerten vor der Weichmacher-Einwirkung gesetzt.

b) Beständigkeit gegenüber Haftkleber:
Auf die gemäß dem Verfahren von (1) dynamisch aufgezeichnete Probe des thermischen Aufzeichnungspapiers wurde je ein Streifen transparentes Tesa-Selbstklebeband (tesafilm® kristall-klar, #57315) und getrennt davon ein Streifen Tesa-Verpackungsklebeband (#04204) unter Vermeiden von Falten und Lufteinschlüssen geklebt. Nach Lagerung bei Raumtemperatur (20 bis 22°C) wurde nach 24 Stunden und nach 7 Tagen die Bilddichte (o.D.) -durch das jeweilige Klebeband hindurch- gemessen und entsprechend der Formel (Gl. 1) in Bezug zu den analog bestimmten Bilddichtewerten der frisch beklebten Muster gesetzt.

$$\%\ verbleibende\ Bilddichte = \left(\frac{Bilddichte\ nach\ Test}{Bilddichte\ vor\ Test}\right) * 100 \qquad (\text{Gl. } 1)$$

Die Streuung der nach (Gl. 1) berechneten %-Werte beträgt ≤±2 Prozentpunkte.

Die Tabellen 3 bis 5 fassen die Auswertung der hergestellten Aufzeichnungsmaterialien zusammen.

Tabelle 3: Bilddichte (optische Dichte bei einer Bestromungsenergie von 0,25 und 0,45 mJ/dot) sowie Starttemperatur (Startpunkt) der Farbentwickler

| Farbentwickler | o.D. (0,25 mJ/dot) | o.D. (0,45 mJ/dot) | Startpunkt (°C) |
|---|---|---|---|
| **I** | 1,16 | 1,22 | 81 |
| **II** | 1,20 | 1,24 | 76 |
| **III** | 1,13 | 1,24 | 76 |
| **IV** | 1,19 | 1,20 | 71 |
| **V** | 1,17 | 1,20 | 76 |
| **VII** | 1,15 | 1,23 | 86 |
| **VIII** | 1,20 | 1,23 | 73 |
| **X** | 1,24 | 1,30 | 82 |
| **XIII** | 1,14 | 1,20 | 79 |
| **XXIII** | 1,16 | 1,26 | 82 |
| **XXIV** | 1,09 | 1,26 | 82 |
| **Z** | 1,19 | 1,24 | 86 |
| **PF201** | 1,19 | 1,23 | 76 |

Tabelle 4: Bilddichte (optische Dichte bei einer Bestromungsenergie von 0,25 und 0,45 mJ/dot) in Abhängigkeit des Substitutionsmusters der Farbentwickler

| Farbentwickler | Relative Stellung der Sulfonamid- u. Harnstoff-Gruppen | o.D. (0,25 mJ/dot) | o.D. (0,45 mJ/dot) |
|---|---|---|---|
| **II** | 1,2 | 1,20 | 1,24 |
| **XIV** | 1,3* | 1,05 | 1,16 |
| **XVII** | 1,4* | 0,55 | 1,20 |
| **III** | 1,2 | 1,13 | 1,24 |
| **XV** | 1,3* | 1,00 | 1,17 |
| **XVIII** | 1,4* | 0,84 | 1,19 |
| **IV** | 1,2 | 1,19 | 1,20 |
| **XVI** | 1,3* | 0,93 | 1,16 |
| **XIX** | 1,4* | 0,57 | 1,19 |
| *: Vergleichsbeispiele | | | |

Tabelle 5: Beständigkeit des Druckbildes der Farbentwickler

| Farbentwickler | Tesa-Klebeband* | | | | Weichmacher-Folie* | |
|---|---|---|---|---|---|---|
| | 24 h | | 7 Tage | | 16 h | |
| | #57315 | #04204 | #57315 | #04204 | R.T. | 40 °C |
| **I** | 83 | 47 | 16 | 14 | 98 | 88 |
| **II** | 74 | 43 | 34 | 16 | 99 | 77 |
| **III** | 71 | 49 | 37 | 19 | 97 | 83 |
| **IV** | 71 | 42 | 32 | 14 | 98 | 89 |
| **V** | 67 | 38 | 18 | 11 | 97 | 68 |
| **VII** | 70 | 40 | 33 | 15 | 98 | 84 |
| **VIII** | 71 | 46 | 23 | 15 | 88 | 67 |
| **X** | 75 | 44 | 25 | 20 | 99 | 90 |
| **XIII** | 74 | 37 | 33 | 16 | 100 | 93 |
| **XXIII** | 92 | 90 | 74 | 65 | 94 | 92 |
| **XXIV** | 91 | 89 | 68 | 61 | 100 | 95 |
| **Z** | 60 | 35 | 12 | 13 | 83 | 17 |
| **PF201** | 73 | 46 | 32 | 16 | 97 | 78 |
| *Prozentuale verbleibende Bilddichte entsprechend Gl. 1 | | | | | | |

Aus vorstehenden Beispielen lässt sich entnehmen, dass das wärmeempfindliche Aufzeichnungsmaterial der vorliegenden Erfindung insbesondere die folgenden vorteilhaften Eigenschaften zeigt:

(1) Das aufgezeichnete Bild der wärmeempfindlichen Aufzeichnungsmaterialien basierend auf den erfindungsgemäßen Farbentwicklern weist Druckdichten (optische Dichten) auf, die vergleichbar jener der Vergleichsmuster aus dem Stand der Technik sind (Tabelle 3).

(2) Die auf Farbentwicklern mit dem erfindungsgemäßen Substitutionsmuster (1,2-Stellung (ortho-Stellung) der

relevanten funktionellen Gruppen) basierenden wärmeempfindlichen Aufzeichnungsmaterialien haben signifikant höhere Druckdichten als die auf Farbentwicklern mit alternativen Substitutionsmustern (1,3- und 1,4-Stellung der relevanten funktionellen Gruppen) basierenden Aufzeichnungsmaterialien. Vgl. **II** mit **XIV** und **XVII, III** mit **XV** und **XVIII** sowie **IV** mit **XVI** und **XIX** (Tabelle 4).

(3) Die Temperatur, ab welcher eine visuell merkliche Vergrauung der erfindungsgemäßen Aufzeichnungsmaterialien eintritt (statischer Startpunkt), erfüllt die Anforderungen an markttaugliche wärmeempfindliche Aufzeichnungsmaterialien (Tabelle 3).

(4) Die Bildbeständigkeit ist nach Einwirken hydrophober Agenzien (Klebstoffe, Weichmacher) besser oder vergleichbar der entsprechenden Leistung der bekannten nicht-phenolischen Farbentwickferstoffen des Standes der Technik (Tabelle 5).

(5) Mit den erfindungsgemäßen Farbentwicklern lässt sich ein in wichtigen anwendungstechnischen Belangen hochwertiges wärmeempfindliches Aufzeichnungsmaterial erhalten. Kein auf bekannten Farbentwicklern basierendes Aufzeichnungsmaterial weist ein vergleichbar ausgewogenes Leistungsprofil über alle geprüften Eigenschaften auf.

## Patentansprüche

**1.** Verbindung der Formel (I),

$$(Ar^1\text{-}SO_2\text{-}NH\text{-})_m\text{-}Y\text{-}(\text{-}NH\text{-}C(O)\text{-}NH\text{-}SO_2\text{-}Ar^2)_n \qquad (I),$$

wobei $Ar^1$ ein unsubstituierter oder substituierter aromatischer Rest, $Ar^2$ ein unsubstituierter oder substituierter Phenyl-Rest, Y eine (m+n)-fach substituierte Benzol- oder Naphthalin-Gruppe und Y derart substituiert ist, dass mindestens eine $Ar^2$-$SO_2$-NH-C(O)-NH-Gruppe in ortho-Position zu mindestens einer $Ar^1$-$SO_2$-NH-Gruppe steht.

**2.** Verbindung nach Anspruch 1, wobei m=1 und n≥1 ist.

**3.** Verbindung nach Anspruch 1 oder 2, wobei n 1 oder 2 ist.

**4.** Verbindung nach mindestens einem der vorangegangenen Ansprüche, wobei $Ar^1$ ein unsubstituierter oder substituierter Phenyl-, ein unsubstituierter oder substituierter 2-Naphthyl-Rest ist.

**5.** Verbindung nach Anspruch 4, wobei $Ar^1$ ein unsubstituierter Phenyl- oder ein einfach substituierter Phenyl-Rest ist.

**6.** Verbindung nach Anspruch 5, wobei der einfach substituierte Phenyl-Rest mit einem $C_1$-$C_5$-Alkyl-, einem Alkenyl-, einem Alkinyl-, einem Benzyl-, eine RO-, einem Halogen-, einem Formyl-, einem ROC-, einem $RO_2C$-, einem CN-, einem $NO_2$-, R-$SO_2$O-, einem RO-$SO_2$-, einem R-NH-$SO_2$-, einem R-$SO_2$NH-, einem R-NH-CO-NH-, einem R-$SO_2$-NH-CO-NH-, einem R-NH-CO-NH-$SO_2$- oder einem R-CO-NH-Rest, wobei R ein $C_1$-$C_5$-Alkyl-, ein Alkenyl-, ein Alkinyl-, ein Phenyl-, ein Tolyl- oder ein Benzyl-Rest, bevorzugt ein Phenyl- oder ein p-Tolyl-Rest ist, substituiert ist.

**7.** Verbindung nach mindestens einem der vorangegangenen Ansprüche, wobei $Ar^2$ ein unsubstituierter Phenyl- oder ein einfach substituierter Phenyl-Rest, insbesondere ein mit einem $C_1$-$C_4$-Alkyl-Rest, besonders bevorzugt mit einem Methyl-Rest substituierter Phenyl-Rest ist.

**8.** Verbindung nach mindestens einem der vorangegangenen Ansprüche, wobei $Ar^1$ ein unsubstituierter Phenyl-Rest oder ein einfach substituierter Phenyl-Rest, $Ar^2$ ein unsubstituierter Phenyl-Rest oder ein einfach substituierter Phenyl-Rest und Y eine (m+n)-fach substituierte Benzol-Gruppe ist.

**9.** Wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, wobei der mindestens eine Farbentwickler die Verbindung der Formel (I) nach mindestens einem der Ansprüche 1 bis 8 ist.

**10.** Wärmeempfindliches Aufzeichnungsmaterial nach Anspruch 9, wobei der mindestens eine Farbbildner ein Farbstoff vom Triphenylmethan-Typ, vom Fluoran-Typ, vom Azaphthalid-Typ und/oder vom Fluoren-Typ, bevorzugt vom

Fluoran-Typ, ist.

**11.** Wärmeempfindliches Aufzeichnungsmaterial nach mindestens einem der Ansprüche 9 und 10, wobei neben der Verbindung der Formel (I) ein oder mehrere weitere nicht-phenolische Farbentwickler vorliegen.

**12.** Wärmeempfindliches Aufzeichnungsmaterial nach mindestens einem der Ansprüche 9 bis 11, wobei die Verbindung der Formel (I) nach mindestens einem der Ansprüche 1 bis 8 in einer Menge von etwa 3 bis etwa 35 Gew.-%, bevorzugt von etwa 10 bis etwa 25 Gew.-%, bezogen auf den gesamten Feststoffgehalt der wärmeempfindlichen Schicht, vorliegt.

**13.** Wärmeempfindliches Aufzeichnungsmaterial nach mindestens einem der Ansprüche 9 bis 12, wobei die wärmeempfindliche farbbildende Schicht eine Harnstoff-Urethan-Verbindung der allgemeinen Formel (II)

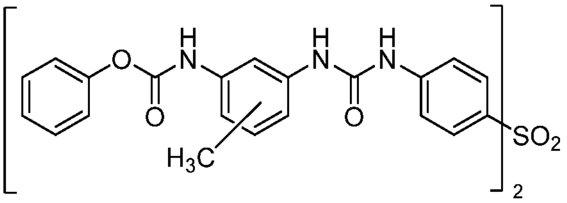

(II)

enthält.

**14.** Verfahren zur Herstellung eines wärmeempfindlichen Aufzeichnungsmaterials nach mindestens einem der Ansprüche 9 bis 13, wobei auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-% , bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min, bevorzugt von mindestens etwa 1000 m/min, ganz besonders bevorzugt von mindestens etwa 1500 m/min, aufgetragen und getrocknet wird.

**15.** Wärmeempfindliches Aufzeichnungsmaterial, erhältlich gemäß dem Verfahren nach Anspruch 14.

## Claims

**1.** A compound of formula (I)

$$(Ar^1\text{-}SO_2\text{-}NH\text{-})_m\text{-}Y\text{-}(\text{-}NH\text{-}C(O)\text{-}NH\text{-}SO_2\text{-}Ar^2)_n \qquad (I),$$

wherein $Ar^1$ is an unsubstituted or substituted aromatic group, $Ar^2$ is an unsubstituted or substituted phenyl group, Y is at least one benzene group or naphthalene group substituted (m+n) times, and Y is substituted in such a way that at least one $Ar^2$-$SO_2$-NH-C(O)-NH group is in the ortho position with respect to at least one $Ar^1$-$SO_2$-NH group.

**2.** The compound according to claim 1, wherein m=1 and n≥1.

**3.** The compound according to claim 1 or claim 2, wherein n is 1 or 2.

**4.** The compound according to at least one of the preceding claims, wherein $Ar^1$ is an unsubstituted or substituted phenyl group, or an unsubstituted or substituted 2-naphthyl group.

**5.** The compound according to claim 4, wherein $Ar^1$ is an unsubstituted phenyl or a monosubstituted phenyl group.

**6.** The compound according to claim 5, wherein the monosubstituted phenyl group is substituted with a $C_1$-$C_5$ alkyl, an alkenyl, an alkynyl, a benzyl, an RO, a halogen, a formyl, an ROC, an $RO_2C$, a CN, an $NO_2$, an R-$SO_2$O, an RO-$SO_2$, an R-NH-$SO_2$, an R-$SO_2$-NH, an R-NH-CO-NH, an R-$SO_2$-NH-CO-NH, an R-NH-CO-NH-$SO_2$ or an R-CO-NH group, wherein R is a $C_1$-$C_5$ alkyl, an alkenyl, an alkynyl, a phenyl, a tolyl or a benzyl group, preferably a

phenyl or a p-tolyl group.

7. The compound according to at least one of the preceding claims, wherein $Ar^2$ is an unsubstituted phenyl group or a monosubstituted phenyl group, especially a phenyl group substituted with a $C_1$-$C_4$ alkyl group, especially preferably a phenyl group substituted with a methyl group.

8. The compound according to at least one of the preceding claims, wherein $Ar^1$ is an unsubstituted phenyl group or a monosubstituted phenyl group, $Ar^2$ is an unsubstituted phenyl group or a monosubstituted phenyl group, and Y is a benzene group substituted (m+n) times.

9. A heat-sensitive recording material, comprising a carrier substrate and a heat-sensitive colour-forming layer, which contains at least one colour former and at least one phenol-free colour developer, wherein the at least one colour developer is the compound of formula (I) according to at least one of claims 1 to 8.

10. The heat-sensitive recording material according to claim 9, wherein the at least one colour former is a dye of the triphenylmethane type, of the fluorane type, of the azaphthalide type and/or of the fluorene type.

11. The heat-sensitive recording material according to at least one of claims 9 and 10, wherein, besides the compound of formula (I), one or more further non-phenolic colour developers are also present.

12. The heat-sensitive recording material according to at least one of claims 9 to 11, wherein the compound of formula (I) according to at least one of claims 1 to 8 is present in an amount of from approximately 3 to approximately 35 % by weight, preferably from approximately 10 to approximately 25 % by weight, in relation to the total solids content of the heat-sensitive layer.

13. The heat-sensitive recording material according to at least one of clams 9 to 12, wherein the heat-sensitive colour-forming layer contains a urea urethane compound of general formula (II)

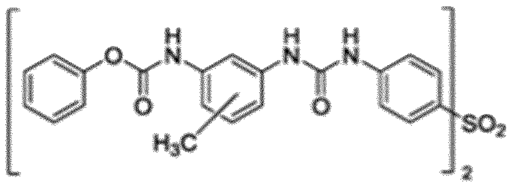

(II) .

14. A method for producing a heat-sensitive recording material according to at least one of claims 9 to 13, wherein an aqueous suspension containing the starting materials of the heat-sensitive colour-forming layer is applied to a carrier substrate and dried, wherein the aqueous suspension to be applied has a solids content of from approximately 20 to approximately 75 % by weight, preferably from approximately 30 to approximately 50 % by weight, and is applied using the curtain-coating process at an operating speed of the coating facility of at least approximately 400 m/min, preferably of at least approximately 1000 m/min, very especially preferably of at least approximately 1500 m/min, and is dried.

15. A heat-sensitive recording material obtainable by the method according to claim 14.

**Revendications**

1. Composé de la formule (I),

$$(Ar^1\text{-}SO_2\text{-}NH\text{-})_m\text{-}Y\text{-}(\text{-}NH\text{-}C(O)\text{-}NH\text{-}SO_2\text{-}Ar^2)_n \qquad (I),$$

dans lequel $Ar^1$ est un radical aromatique non substitué ou substitué, $Ar^2$ est un radical phényle non substitué ou substitué, Y est un groupe benzène ou naphtalène substitué (m+n) fois et Y est substitué de telle manière qu'au moins un groupe $Ar^2$-$SO_2$-NH-C(O)-NH se trouve dans une position ortho par rapport à au moins un groupe $Ar^1$-$SO_2$-NH.

2. Composé selon la revendication 1, dans lequel m = 1 et $n \geq 1$.

3. Composé selon la revendication 1 ou 2, dans lequel n est 1 ou 2.

4. Composé selon au moins l'une quelconque des revendications précédentes, dans lequel $Ar^1$ est un radical phényle non substitué ou substitué, un radical 2-naphtyle non substitué ou substitué.

5. Composé selon la revendication 4, dans lequel $Ar^1$ est un radical phényle non substitué ou un radical phényle substitué une fois.

6. Composé selon la revendication 5, dans lequel le radical phényle substitué une fois est substitué par un radical alkyle en $C_1$-$C_5$, un radical alcényle, un radical alcynyle, un radical benzyle, un radical RO, un radical halogène, un radical formyle, un radical ROC, un radical $RO_2C$, un radical CN, un radical $NO_2$, un radical R-$SO_2$O, un radical RO-$SO_2$, un radical R-NH-$SO_2$, un radical R-$SO_2$NH, un radical R-NH-CO-NH, un radical R-$SO_2$-NH-CO-NH, un radical R-NH-CO-NH-$SO_2$ ou un radical R-CO-NH, dans lequel R est un radical alkyle en $C_1$-$C_5$, un radical alcényle, un radical alcynyle, un radical phényle, un radical tolyle ou un radical benzyle, de manière préférée un radical phényle ou un radical p-tolyle.

7. Composé selon au moins l'une quelconque des revendications précédentes, dans lequel $Ar^2$ est un radical phényle non substitué ou un radical phényle substitué une fois, en particulier un radical alkyle en $C_1$-$C_4$, de manière particulièrement préférée est un radical phényle substitué par un radical méthyle.

8. Composé selon au moins l'une quelconque des revendications précédentes, dans lequel $Ar^1$ est un radical phényle non substitué ou un radical phényle substitué une fois, $Ar^2$ est un radical phényle non substitué ou un radical phényle substitué une fois et Y est un groupe benzène substitué (m + n) fois.

9. Matériau d'enregistrement thermosensible, comprenant un substrat de support ainsi qu'une couche chromogène thermosensible contenant au moins un agent chromogène et au moins un révélateur de couleur non phénolique, dans lequel l'au moins un révélateur de couleur est le composé de la formule (I) selon au moins l'une quelconque des revendications 1 à 8.

10. Matériau d'enregistrement thermosensible selon la revendication 9, dans lequel l'au moins un agent chromogène est un colorant du type triphénylméthane, du type fluorane, du type azaphthalide et/ou du type fluorène, de manière préférée du type fluorane.

11. Matériau d'enregistrement thermosensible selon au moins l'une quelconque des revendications 9 et 10, dans lequel un ou plusieurs révélateurs de couleur non phénoliques sont présents outre le composé de la formule (I).

12. Matériau d'enregistrement thermosensible selon au moins l'une quelconque des revendications 9 à 11, dans lequel le composé de la formule (I) selon au moins l'une quelconque des revendications 1 à 8 est présent en une quantité d'environ 3 à environ 35 % en poids, de manière préférée d'environ 10 à environ 25 % en poids, par rapport à la teneur totale en matières solides dans la couche thermosensible.

13. Matériau d'enregistrement thermosensible selon au moins l'une quelconque des revendications 9 à 12, dans lequel la couche chromogène thermosensible contient un composé à base d'urée-uréthane de la formule générale (II)

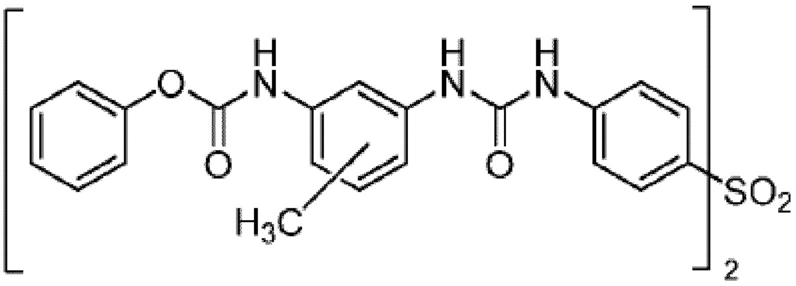

(II)

14. Procédé de fabrication d'un matériau d'enregistrement thermosensible selon au moins l'une quelconque des revendications 9 à 13, dans lequel une suspension aqueuse contenant les matériaux de départ de la couche chromogène thermosensible est appliquée et est séchée sur un substrat de support, dans lequel la suspension d'application aqueuse présente une teneur en matières solides d'environ 20 à environ 75 % en poids, de manière préférée d'environ 30 à environ 50 % en poids, et est appliquée et séchée avec le procédé d'enduction à rideau à une vitesse de fonctionnement de la coucheuse d'au moins 400 m/min, de manière préférée d'au moins environ 1000 m/min, de manière tout particulièrement préférée d'au moins environ 1500 m/min.

**15.** Matériau d'enregistrement thermosensible pouvant être obtenu selon le procédé selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 2006004197 A1
- WO 0172527 A1
- EP 0526072 A1
- WO 0035679 A1
- EP 0535887 A1
- EP 0542556 A1
- EP 0604832 B1
- EP 0620122 A1
- EP 1044824 A2
- JP H0664335 B
- JP H0958242 B
- JP H11263067 B
- DE 10196052 T1

### In der Beschreibung aufgeführte Nicht-Patentliteratur

- **V. MILATA ; J. SALO.** *Org. Prep. Proceed. Int.,* 1999, vol. 31 (3), 347
- **V. MILATA.** Reaktionsschema 3. *Org. Prep. Proceed. Int.,* 1999, vol. 31 (3), 347